# EUROPEAN PATENT SPECIFICATION

(11) **EP 4 412 521 B1**
(45) Date of publication and mention of the grant of the patent: **05.11.2025**
(21) Application number: 22790454.7
(22) Date of filing: 16.09.2022
(51) Int. Cl.: A61B 5/145, A61B 5/00

(54) **APPARATUS FOR TRANSPORTING SWEAT DROPLETS**
VORRICHTUNG ZUM TRANSPORT VON SCHWEISSTROPFEN
APPAREIL DE TRANSPORT DE GOUTTELETTES DE SUEUR

(30) Priority: 04.10.2021 EP 21200652
(43) Date of publication of application: 14.08.2024
(73) Proprietor: Koninklijke Philips N.V., 5656 AG Eindhoven (NL)
(72) Inventor: PELSSERS, Eduard Gerard Marie, 5656 AG Eindhoven (NL); VAN LIESHOUT, Ron Martinus Laurentius, 5656 AG Eindhoven (NL); VERBEEK, Gilbert Martinus, 5656 AG Eindhoven (NL)
(74) Representative: Philips Intellectual Property & Standards
(86) International application number: PCT/EP2022/075717
(87) International publication number: WO 2023/057188

(56) References cited:
- WO-A1-2021/074010
- WO-A2-2009/052123

## Description

### FIELD OF THE INVENTION

This invention relates to an apparatus for transporting sweat droplets.

### BACKGROUND OF THE INVENTION

Non-invasive, semi-continuous and prolonged monitoring of biomarkers that indicate disease/health status and well-being is in demand for monitoring, for example, dehydration, stress, sleep, children's health and in perioperative monitoring.

Sweat, tear fluid and saliva may all be obtained non-invasively. Sweat is a particularly accessible biofluid, and is a rich source of information relating to the physiology and metabolism of a subject.

Some examples of clinical relevant components of sweat are Na⁺, Cl⁻ and/or K⁺ to monitor dehydration, lactate as an early warning for inflammation (which is relevant to sepsis), glucose for diabetics and neonates, and cortisol in relation to sleep apnea and stress monitoring.

Continuous monitoring of high-risk patients, such as those with serious chronic conditions, pre- or post-operative patients, and the elderly, using sweat biomarker monitoring devices can provide higher quality diagnostic information than regular biomarker spot checks as normally done by repeatedly drawing multiple blood samples. Such continuous monitoring may be in a hospital setting or elsewhere. Human sweat alone or as mixture with sebum lipids may be an easily accessible source for biomarker measurements in wearable on-skin devices. For instance, cholesterol is an important biomarker associated with elevated risk in development of cardiovascular diseases. Inflammatory markers or cytokines, such as interleukins (e.g. TNF-a, IL-6) play an important role in the immune response and detection or disease monitoring of joint damage in rheumatoid and psoriatic arthritis, and bowel disease.

Persons in a sedentary state, such as hospital patients, have a minimal sweat rate and there is therefore a significant delay between sweat excretion and biomarker detection, which can prevent timely monitoring and early warning of any impending complication. The concentration of particular relevant biomarkers is sweat rate dependent and therefore sweat rate per gland has to be assessed for a clinically relevant interpretation. Conventional sweat sensing solutions have limited application since they require the monitored person to be engaged in exercise, and tend to use rather complex microfluidics and sensors to determine the sweat rate.

WO 2018/125695 A1 discloses wearable sweat biosensing devices with active sweat sampling. An active method is described for transporting sweat which utilizes the electromechanical effect of electrowetting. Electrowetting plates comprise a hydrophobic dielectric layer (e.g., Teflon) covering electrodes. A sweat coupling "wicking" component made of a hydrophilic material permits sweat from the skin surface to slowly diffuse over time to the electrowetting plates, whereupon the sweat is transported via the electromechanical effect. This approach is very time consuming, and may be ineffective for small sweat volumes due to evaporation. Moreover, the technique entails mixing of sweat received from the skin at different times, which is undesirable for reliable semi-continuous biomarker measurements.

WO 2021/074010 A1 discloses an apparatus for transporting sweat droplets to a sensor. The apparatus comprises a chamber for filling with sweat. The chamber has an inlet lying adjacent the surface of the skin, which inlet permits sweat to enter and fill the chamber. The chamber has an outlet from which a sweat droplet protrudes once the chamber has been filled. The apparatus further comprises a fluid transport assembly which is designed to enable the sweat droplet protruding from the outlet to become detached from the outlet of the chamber. The sweat droplet is subsequently transported by the fluid transport assembly to the sensor. Once the protruding droplet has been released from the outlet, the outlet is made available for a subsequent sweat droplet to protrude therefrom upon further filling of the chamber. The released sweat droplet is transported via the fluid transport assembly at least as fast as the subsequent sweat droplet protrudes from the outlet such that the respective sweat droplets do not contact each other before reaching the sensor. Thus, the apparatus supplies sweat to the sensor in a dropwise manner.

As noted above, it has been demonstrated in the literature that small droplets can be transported by electrowetting. Further, droplets can split and merge by utilizing electrowetting. Complex droplet manipulation of several droplets with electrowetting has been shown on a matrix of electrodes. When many electrodes are involved an active matrix driver layer has been used below the electrode matrix layer to reduce the final number of electrical connection to the control electronics of a device. This active matrix driver layer controls each electrode individually. However, when a wearable disposable patch format is in need, the costs for such a set-up may limit the application space.

### SUMMARY OF THE INVENTION

The invention is defined by the independent claim 1. The dependent claims define advantageous embodiments.

According to an aspect there is provided an apparatus for transporting sweat droplets, the apparatus comprising: a plurality of chambers defined in a substrate, each of the chambers having an inlet for receiving sweat from the skin, and an outlet delimited by a surface of the substrate and arranged such that a sweat droplet forms and protrudes therefrom following filling of the chamber with sweat, wherein the outlets are radially grouped around a central portion of the surface of the substrate to define at least first, second and third groups of chambers; and an electrowetting arrangement comprising: a plurality of electrodes, each electrode being electrically chargeable and dischargeable, wherein the electrowetting arrangement comprises first, second, and third limbs of electrodes configured to permit transport of the sweat droplets from the first group, in the case of the first limb, to or towards a central region, similarly centrally from the second group, in the case of the second limb and similarly centrally from the third group, in the case of the third limb by charging and discharging of the electrodes; and a conductive trace arrangement arranged to electrically connect electrodes of the first, second, and third limbs to each other.

The radial grouping of the outlets around a central portion of the surface of the substrate may mean that the outlets of each of the groups, and in particular the first, second and third groups, are arranged in a radial segment defined between two (e.g. notional) radii extending from the central portion, and a portion of a (e.g. notional) boundary, for example circumference, circumscribing the plurality of chambers so that each of the plurality of chambers are located within the boundary.

The term "limb" may refer to an overall pathway of electrodes which carries the sweat droplets from the outlets of the respective group to or towards the central region. Each limb may include one or more series of electrodes, as described herein below.

The conductive trace arrangement can be regarded as providing electrical connections with and between the electrodes in a plane or level of the apparatus, with the electrodes being thereby connected in this plane or level by the conductive trace arrangement. The conductive trace arrangement is thus distinct from connections made between different planes, or between different levels, of the apparatus, such as Vertical Interconnection Access (VIA) connections. In other words, a VIA is an electrical connection between layers in a physical electronic circuit that goes through the plane of one or more adjacent layers.

By the first, second and third limbs transporting the sweat droplets centrally from the first, second and third groups respectively, the apparatus may be particularly suitable for incorporation in, for instance, a wearable sweat sensing device, e.g. in the form of a wearable patch.

In such a wearable sweat sensing device, one or more sensors are preferably arranged in the abovementioned central region of the apparatus to or towards which the sweat droplets are transported via (at least) the first, second and third limbs. The arrangement of the limbs, which in at least some embodiments can be regarded as a radial configuration of limbs, may assist to minimize the transportation distance from the outlet(s) positioned farthest from the central region to the sensor(s). This may, in turn, assist to minimize evaporation of sweat droplets during their transportation to the sensor(s).

Moreover, by the conductive trace arrangement being arranged to electrically connect electrodes of the first, second, and third limbs to each other, the physical design of the apparatus can be simplified and/or made more robust, e.g. since fewer vertical interconnect access (VIA) connections may be required and/or the use of matrix driver layer to control the charging and discharging of the electrodes may be obviated. Thus, the complexity of the manufacturing process, costs and sources of errors / breakdowns are reduced.

The plurality of electrodes comprises a plurality of sets of electrodes, wherein each set comprises electrodes from the first, second and third limbs which are connected to each other.

For each set, a first conductive trace included in the conductive trace arrangement is configured to connect one of the electrodes of the first limb to a corresponding electrode of the second limb, and a second conductive trace included in the conductive trace arrangement is configured to connect said corresponding electrode of the second limb (connected to the electrode of the second limb) to a corresponding electrode of the third limb.

The first limb may comprise at least one series of first electrodes, with each first electrode of the at least one series being configured to release and/or transport one of the sweat droplets protruding from a respective outlet in the first group. The sweat droplet pathway defined by the series of first electrodes may be regarded as a "level zero track".

The first limb may comprise at least one further series of first electrodes configured to receive sweat droplets from each series of the at least one series of first electrodes, and transport the sweat droplets away, for example centrally away, from the at least one series of first electrodes. The sweat droplet pathway defined by the further series of first electrodes may be regarded as a "level one track".

There may be no chambers, and hence no outlets, associated with the electrodes of the further series. Thus, the electrodes of the further series may be solely employed for transporting sweat droplets, e.g. to or towards at least one sensor.

In some embodiments, the first limb comprises an additional series of first electrodes, and the first limb comprises a plurality of said further series of first electrodes. In such embodiments, the additional series of first electrodes may be configured to receive sweat droplets from each of the plurality of further series of first electrodes, and transport said sweat droplets away, for example centrally away, from the plurality of further series of first electrodes. The additional series of first electrodes can be regarded as a level two track.

There may be no chambers, and hence no outlets, associated with the electrodes of the additional series. Thus, the electrodes of the additional series may be solely employed for transporting sweat droplets, e.g. to or towards at least one sensor.

Alternatively or additionally, the second limb comprises at least one series of second electrodes, with each second electrode of the at least one series being configured to release and/or transport one of said sweat droplets protruding from a respective outlet in the second group. The sweat droplet pathway defined by the series of second electrodes may be regarded as a level zero track.

The second limb may comprise at least one further series of second electrodes configured to receive sweat droplets from each series of the at least one series of second electrodes, and transport said sweat droplets away, for example centrally away, from the at least one series of second electrodes. The sweat droplet pathway defined by the further series of second electrodes may be regarded as a level one track.

In some embodiments, the second limb comprises an additional series of second electrodes, and the second limb comprises a plurality of said further series of second electrodes. In such embodiments, the additional series of second electrodes may be configured to receive sweat droplets from each of the plurality of further series of second electrodes, and transport said sweat droplets away, for example centrally away, from the plurality of further series of second electrodes. The additional series of second electrodes can be regarded as a level two track.

Alternatively or additionally, the third limb may comprise at least one series of third electrodes, with each third electrode of the at least one series being configured to release and/or transport one of said sweat droplets protruding from a respective outlet in the third group. The sweat droplet pathway defined by the series of third electrodes may be regarded as a level zero track.

The third limb may comprise at least one further series of third electrodes configured to receive sweat droplets from each series of the at least one series of third electrodes, and transport said sweat droplets away, for example centrally away, from the at least one series of third electrodes. The sweat droplet pathway defined by the further series of third electrodes may be regarded as a level one track.

**In** some embodiments, the third limb comprises an additional series of third electrodes, and the third limb comprises a plurality of said further series of third electrodes. **In** such embodiments, the additional series of third electrodes may be configured to receive sweat droplets from each of the plurality of further series of third electrodes, and transport said sweat droplets away, for example centrally away, from the plurality of further series of third electrodes. The additional series of third electrodes can be regarded as a level two track.

**In** some embodiments, the at least one series of first electrodes comprises a plurality of series of first electrodes, with each of the plurality of series of first electrodes intersecting with one further series of said at least one further series of first electrodes at different positions relative to each other along said one further series of first electrodes.

Thus, the level zero tracks may move sweat droplets onto several locations of the level one track. This may provide a spatially efficient arrangement which renders the apparatus particularly suitable for incorporation into, for instance, a wearable patch.

In some embodiments, the sweat droplets may be subsequently transported from such level one tracks (with which the level zero tracks intersect at different positions) onto a level two track, e.g. again exiting on several locations of the level two track, and finally the level two tracks may, for instance, transport droplets to or through each a sweat rate sensor and towards a central region at or proximal to which a biosensor may be located.

More generally, in some embodiments the at least one further series of first electrodes comprises a plurality of further series of first electrodes, with each of the plurality of further series of first electrodes intersecting with the additional series of first electrodes at different positions relative to each other along the additional series of first electrodes.

By optimizing the density of the chambers in this manner, a smaller wearable patch incorporating the apparatus can be placed on the skin, whilst maintaining sweat collection performance.

Alternatively or additionally, the at least one series of second electrodes may comprise a plurality of series of second electrodes, with each of the plurality of series of second electrodes intersecting with one further series of said at least one further series of second electrodes at different positions relative to each other along said one further series of second electrodes.

In some embodiments, the at least one further series of second electrodes comprises a plurality of further series of second electrodes, with each of the plurality of further series of second electrodes intersecting with the additional series of second electrodes at different positions relative to each other along the additional series of second electrodes.

Alternatively or additionally, the at least one series of third electrodes may comprise a plurality of series of third electrodes, each of the plurality of series of third electrodes intersecting with one further series of said at least one further series of third electrodes at different positions relative to each other along said one further series of third electrodes.

In some embodiments, the at least one further series of third electrodes comprises a plurality of further series of third electrodes, with each of the plurality of further series of third electrodes intersecting with the additional series of third electrodes at different positions relative to each other along the additional series of third electrodes.

In some embodiments, the apparatus may comprise four, five, six, seven, eight, nine, ten or more of the above-defined limbs.

In some embodiments, each limb is symmetrical due to having a mirror plane extending along a radial centerline of the limb.

In embodiments, in which each limb comprises the mirror plane, conductive traces of the conductive trace arrangement may change direction at said radial centerline of each limb. This may assist to reduce the number of VIAs.

Each direction change at the radial centerline of each limb may result in the conductive trace arrangement appearing star-shaped in plan.

More generally, the apparatus may comprise a further substrate on and/or in which the electrowetting arrangement and the conductive trace arrangement are arranged.

In some embodiments, the further substrate comprises a first radially extending edge and a second radially extending edge, with the limbs being arranged on and/or in the substrate between the first and second radially extending edges.

In certain embodiments, the conductive traces of the conductive trace arrangement extend from proximal to the first radially extending edge around to proximal to the second centrally extending edge.

VIAs may, for example, be provided proximal to the first and/or second radially extending edges. In certain embodiments, the apparatus may not include any VIAs, e.g. due to the process employed for fabricating the ends of the electrical traces proximal to the first and second radially extending edges.

The apparatus may comprise at least one of a first sweat rate sensor, a second sweat rate sensor, and a third sweat rate sensor. Such a first sweat rate sensor may be arranged downstream of the outlets of the first group and configured to determine a sweat rate from sweat droplets transported by the first limb. Similarly, the second sweat rate sensor may be arranged downstream of the outlets of the second group and configured to determine a sweat rate from sweat droplets transported by the second limb, and the third sweat rate sensor may be arranged downstream of the outlets of the third group and configured to determine a sweat rate from sweat droplets transported by the third limb.

More generally, the apparatus may include a sweat rate sensor per limb.

The apparatus may comprise a biosensor configured to determine a sweat analyte concentration. The biosensor may be arranged to receive sweat droplets transported thereto by the first, second and third limbs. The biosensor may thus be centrally positioned, e.g. in or proximal to the central region, such as at or proximal to the central region located on the abovementioned further substrate, in order to receive the sweat droplets transported thereto via the limbs.

In some embodiments, the apparatus comprises a sweat collector, such as an absorber/absorbent pad, arranged downstream of one of more sensors. In particular, such a sweat collector may be arranged downstream of the biosensor when such a biosensor is included in the apparatus.

Such a sweat collector may, for example, be arranged in a space, e.g. a radial segment space, defined between the above-described first and second radially extending edges.

The apparatus may comprise an electric field generator for charging and discharging each of the electrodes of the electrowetting arrangement such as to transport each said sweat droplet. The electric field generator may be connected to the electrodes via the conductive trace arrangement.

According to another aspect there is provided a wearable device, such as a wearable patch, comprising the apparatus described above.

In some embodiments, the wearable device comprises an attachment arrangement configured to enable attachment of the apparatus to a body part such that said inlets receive sweat from the skin of the body part.

### BRIEF DESCRIPTION OF THE DRAWINGS

Embodiments of the invention are described in more detail and by way of non-limiting examples with reference to the accompanying drawings, wherein:
Fig. 1 shows a first view of part of an exemplary apparatus for transporting sweat droplets;
Fig. 2 shows a second view of the part of the apparatus shown in Fig. 1;
Fig. 3 shows a third view of the part of the apparatus shown in Fig. 1;
Fig. 4 shows sweat droplets being transported using the apparatus shown in Figs. 1-3;
Fig. 5 shows electrical connections of the electrodes shown in Figs. 3 and 4;
Fig. 6 shows an apparatus for transporting sweat droplets according to a first example;
Fig. 7 shows an apparatus for transporting sweat droplets according to a second example;
Fig. 8 shows an apparatus for transporting sweat droplets according to a third example;
Figs. 9A-E provide views of an apparatus for transporting sweat droplets according to a fourth example; and
Figs. 10A-D schematically depict an exemplary manufacturing process.

### DETAILED DESCRIPTION OF THE EMBODIMENTS

Sampling of sweat over time, for determining the sweat rate per gland and for determining biomarker concentration in sweat, is hampered by the small sweat rate especially in the case of persons in sedentary state; which can be as low as 0.1-0.3 nl per minute for one eccrine sweat gland. It has been shown that a 'discretized sweat collection', i.e. sweat collection of discrete sweat droplets, using electrowetting on dielectrics (EWOD) creates a unique way to optimize the sweat liquid flow towards a sweat rate sensor and a biosensor; the latter determining the concentration of a biomarker. In the literature often methods are described that collect sweat via a continuous flow of sweat, however, even to fill a small microfluidic chamber positioned on the human skin with a height of 50 µm can take tens of hours for a person in sedentary state. Hence, these methods are limited to persons with high sweat rate such as athletes and laborers doing heavy exercise. The presented 'discretized sweat collection' method can collect sweat in tens of seconds and typically uses another 10-30 seconds for transport to the sensors.

However, especially in sedentary state the active gland density is rather low: typically, 10 active glands per cm². Note that this number does vary from individual to individual and varies per body location. To have sufficient sweat to serve the measurement of the biomarker concentration, the sampling of one cm² of skin is desired. However, to allow sweat collection in the order of seconds the sweat collection chambers have to be very small in height and diameter. Due to the small diameter of typically 10 to 100 µm, and more typically between 20 to 60 µm, many of these chambers may be required, such as in the order of ten thousand. Each sweat droplet emerging from a hole of such a cylindrical chamber has to be transported to the sensors. With an EWOD trajectory these droplets can be transported in the order of a few tens of seconds to the sensors. However, each chamber has to be affiliated to an electrode, to enable transport by EWOD. Consequently, the EWOD trajectory requires a large number of electrodes, also in the order of ten thousand.

This increase of scale and complexity on the fluid side has an impact on the increase of complexity in terms of the electrical connections required for the EWOD trajectory. The need for droplet transport from the sweat collection chambers towards a centralized biosensor whilst dealing with a relatively large number of collection chambers where the sweat sample is discretized, i.e the sweat sample comprises discrete sweat droplets, creates a large number of electrical paths that need to cross each other. In order to achieve this, a large number of VIAs may be required and they add complexity, cost and generate a source of error/breakdown. Without optimization, the number of VIAs for a EWOD design, serving about 10.000 tiny collection chambers, may be large, e.g. in the order of several thousands. The alternative of employing a matrix driver layer may add to the cost and complexity and may be incompatible with the device being wearable.

Provided is an apparatus for transporting sweat droplets, e.g. to one or more sensors. The apparatus comprises a plurality of chambers for filling with sweat. Each chamber is defined in a substrate, and has an inlet lying adjacent the surface of the skin. The inlet permits sweat to enter and fill the chamber. Each chamber also has an outlet, delimited by a surface of the substrate, from which a sweat droplet protrudes once the chamber has been filled. The outlets are radially grouped around a central portion of the surface of the substrate to define at least first, second and third groups of chambers. The apparatus further comprises an electrowetting arrangement comprising a plurality of electrodes. Each of the electrodes is electrically chargeable and dischargeable. The electrowetting arrangement comprises at least first, second, and third limbs of electrodes configured to permit transport of the sweat droplets centrally from the first group in the case of the first limb, centrally from the second group in the case of the second limb, and centrally from the third group in the case of the third radial portion by charging and discharging of the electrodes. The electrowetting arrangement also includes a conductive trace arrangement arranged to electrically connect electrodes of the first, second, and third limbs to each other.

By the first, second and third limbs transporting the sweat droplets centrally from the first, second and third groups respectively, the apparatus may be particularly suitable for incorporation in, for instance, a wearable sweat sensing device, e.g. in the form of a wearable patch.

In such a wearable sweat sensing device, one or more sensors is or are preferably arranged in the central region of the apparatus to or towards which the sweat droplets are transported via the first, second and third limbs. The arrangement of the limbs, which in at least some embodiments can be regarded as a radial configuration of limbs, may assist to minimize the transportation distance from the outlet(s) positioned farthest from the central region to the sensor(s). This may, in turn, assist to minimize evaporation of sweat droplets during their transportation to the sensor(s).

Moreover, by the conductive trace arrangement being arranged to electrically connect electrodes of the first, second, and third limbs to each other, the physical design of the apparatus may be simplified and/or made more robust, e.g. since fewer vertical interconnect access (VIA) connections may be required to avoid short circuiting issues and/or the use of matrix driver layer to control the charging and discharging of the electrodes may be obviated.

Examples of VIA manufacturing processes include through-glass VIAs (TGVs) in glass substrates, redistribution layer (RDL) circuit patterning on glass, and channels and shaped VIA-forming in glass substrates. A VIA is an electrical connection between layers in a physical electronic circuit that goes through the plane of one or more adjacent layers. If well made, PCB VIAs may primarily fail due to differential expansion and contraction between the copper plating and the PCB in the out of plane direction (Z). This differential expansion and contraction may induce cyclic fatigue in the copper plating, eventually resulting in crack propagation and an electrical open. Various design, material, and environmental parameters will influence the rate of this degradation. This is especially relevant for wearable devices. Hence it is desirable to minimize the number of VIAs included in the electrowetting arrangement.

It is noted that the dropwise or discretized flow of sweat offers several unique benefits with respect to continuous flow. The delay between excretion of sweat and the actual determination of the biomarker concentration may be reduced, e.g. from typically 1-2 hours to about 10-15 minutes for subjects in a sedentary state. The capability of handling minute amounts of sweat and being able to transport this relatively rapidly to the sensor may enable, in the case of the sensor comprising a biomarker sensor, biomarker concentrations to be determined, even when subjects are in a sedentary state. Moreover, the sweat rate may be more straightforwardly determined, e.g. using simpler sensors, when the sweat is provided as discrete sweat droplets rather than as a continuous flow.

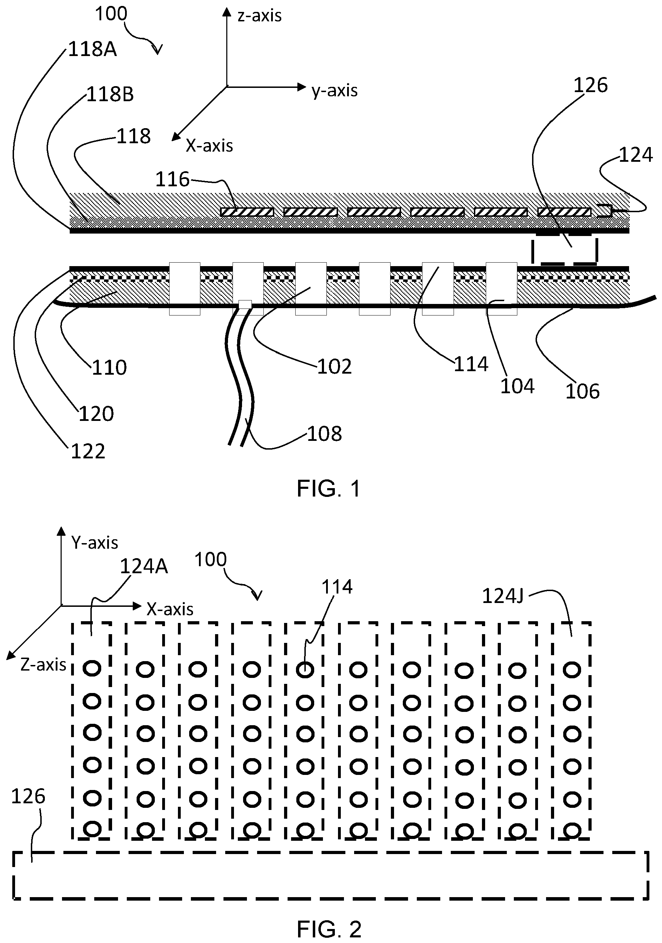

Fig. 1 schematically depicts, in cross-sectional view, part of an apparatus 100 according to an example. The apparatus 100 comprises a plurality of chambers 102, with each of the chambers 102 having an inlet 104. The inlet 104 receives sweat from the skin 106. As shown in Fig. 1, the inlet 104 may be disposed adjacent to a surface of the skin 106.

The inlet 104 is shown proximal to a sweat gland 108. In this case, the sweat excreted by the sweat gland 108 enters and fills the chamber 102 via the inlet 104. As shown in Fig. 1, the apparatus 100 may comprise a substrate 110 which is attached to the surface of the skin 106. In the depicted example, a lower surface of the substrate 110 is in direct contact with the surface of the skin 106. In this case, the chamber 102 takes the form of an aperture delimited by the substrate 110. As shown in Fig. 1, each of the chambers 102 is oriented parallel with the z-axis, but other orientations are also possible.

The substrate 110 may be formed of any suitable material, e.g. a polymer, capable of being disposed on the skin. For example, the substrate 110 may have at least a degree of flexibility so as to enable conformal application to the surface of the skin 106. More rigid substrates 110 may also be contemplated, providing the inlet 104 can receive sweat from the skin 106.

In order to collect sweat from a subject, the substrate 110 may, for instance, be adhered to the surface of the skin 106 using a suitable adhesive. Alternatively, the substrate 110 may be held against the surface of the skin 106 by fastenings, e.g. straps, for attaching the substrate 110 to the body of the subject.

More generally, the apparatus 100 may be included in a wearable device, such as a wearable patch.

In some embodiments, the wearable device comprises an attachment arrangement, such as the above-described adhesive and/or fastenings, configured to enable attachment of the apparatus 100 to a body part such that said inlets 104 receive sweat from the skin 106 of the body part.

The respective areas of the inlet 104 and the outlet 114 may be selected to ensure efficient filling of the chamber 102 and sweat droplet formation over a range of sweat rates. In some examples, the inlet 104 and the outlet 114 have selected fixed dimensions for this purpose. In a preferred example, each chamber 102 is dimensioned to fill up with sweat at least within 10-15 minutes. The formation of the hemispherical sweat droplet following filling of the chamber 102 preferably occurs typically within 10 seconds at relatively low sweat rate, e.g. 0.2 nl/min/gland.

In an embodiment, each inlet 104 of the plurality of chambers 102 is dimensioned to receive sweat from, on average, 0.1 to 1 active sweat glands. This may assist the apparatus 100 to be used for determination of the sweat rate per sweat gland.

Each inlet 104 may, for example, have an area between 0.005 mm² to 20 mm². The inlet area may be selected according to the other dimensions of the chambers 102, and the number of chambers 102 included in the apparatus 100.

Once the chamber 102 has been filled with sweat, a sweat droplet protrudes from an outlet 114 of the chamber 102. In the example shown in Fig. 1, the outlet 114 is delimited by an upper surface of the substrate 110, and a hemispherical sweat droplet forms on top of the outlet 114 once the chamber 102 has been filled with sweat.

The volume of the chamber 102 may be minimized in various ways in order to minimize the time required to fill the chamber 102 with sweat. Such modifications may be, for instance, to the shape of each of the chambers 102, e.g. using a tapering shape which narrows in the direction of the outlet 114, inclusion of a porous material in the chambers 102, and so on.

The apparatus 100 comprises an electrowetting arrangement which is arranged to transport each sweat droplet protruding from a respective outlet 114. The electrowetting arrangement comprises a plurality of electrically chargeable and dischargeable electrodes 116.

In the non-limiting example shown in Fig. 1, the electrodes 116 are arranged in a further substrate 118 which opposes the upper surface of the substrate 110, which upper surface delimits the outlets 114.

The further substrate 118 may comprise a hydrophobic layer 118A for directly contacting the sweat droplets. Adjacent this hydrophobic layer 118A is at least one dielectric layer 118B, e.g. one, two or more dielectric layer(s) 118B. The electrodes 116 of the electrowetting arrangement are positioned adjacent the at least one dielectric layer 118B. Thus, the at least one dielectric layer 118B is interposed between the electrodes 116 and the hydrophobic layer 124A.

A sweat droplet that is partially in contact with such hydrophobic layer, will encounter a driving force and the counter forces, viscous drag and contact angle hysteresis when an electrode 116 is charged beneath the surface where the droplet is partially overlapping with. The driving force is created by a surface energy gradient which promotes the motion of the sweat droplet whereas viscous drag and contact angle hysteresis opposes the motion of the sweat droplet. The contact angle hysteresis acts as a resistant force to the movement, trying to retain the sweat droplet in its static position. The sweat droplet accelerates under the resultant force of these opposing forces.

Charging of an electrode 116, directly above the hydrophobic layer 118A, will lower the contact angle between the sweat droplet and this layer; causing a pulling force for the sweat droplet to move completely to this surface. Although the surface of hydrophobic layer does not necessarily change, one can speak of a layer switching from a hydrophobic to a hydrophilic property. The sweat droplet may correspondingly migrate onto a portion of the hydrophobic layer adjacent the charged electrode 116. Subsequent discharge of the charged electrode 116 and charging of the subsequent electrode 116 in the transport direction may cause the sweat droplet to migrate to a portion of the hydrophobic layer 118A adjacent the subsequent electrode 116, and so on. This sequence may be regarded as an "electrowetting wave".

Thus, by coating the electrodes 116 with a hydrophobic layer 118A, such as a hydrophobic layer 118A comprising or consisting of a chloropolymer, for example a fluoropolymer, for example CYTOP^{®} & Fluoropel, and charging/discharging of the electrodes 116 may permit switching of the hydrophobicity properties of the hydrophobic layer 118A. The layer 118B may be a parylene layer but can also be a layered coating of various substances, such as sputtering tantalum pentoxide or silicon-nitride on the electrode, coated with paralene and finally layer 118A is applied.

The use of an electrowetting arrangement in order to transport/migrate sweat droplets may offer relatively rapid migration and precise control over the transport, e.g. velocities, of the sweat droplets. The propagation of the electrowetting wave may, in principle, be applied to transport sweat droplets over relatively long distances.

An electric field generator (not visible) connected to the electrodes 116 may be used to charge/discharge the electrodes 116. The electric field generator may, for example, be included in the apparatus 100, and in particular the electrowetting arrangement. **In** other examples, the electric field generator is supplied separately from the apparatus 100.

Controlled local voltages may be employed to generate multiple electrowetting waves. Such electrowetting waves may affect transport/migration of the sweat droplet towards or to, and optionally through, one or more sensors (not visible in Fig. 1).

In addition to the electrodes 116, the electrowetting arrangement may include a ground electrode 120. In the non-limiting example shown in Fig. 1, such a ground electrode takes the form of or comprises a conductive layer 120, for example an indium tin oxide layer, disposed beneath a hydrophobic layer 122. The hydrophobic layer 122 is exposed to the gap provided between the substrate 110 and the further substrate 118, as shown.

In at least some embodiments, the detachment or release of the sweat droplet from the outlet 114 may occur at the moment that the sweat droplet reaches a certain diameter.

In the non-limiting example shown in Fig. 1, the positioning of the further substrate 118 may enable control to be exerted over the volume of each sweat droplet. This may be achieved, for instance, by the further substrate 118 being separated from the substrate 110 by a defined distance. Such a defined distance can, for example, be provided by spacer elements arranged between the substrate 110 and the further substrate 118. The sweat droplet may increase in size until it makes contact with the further substrate 118. In practice, when the sweat droplet contacts the further substrate 118, the sweat droplet may become detached by "jumping" over to the further substrate 118.

Alternatively or additionally, sweat droplet detachment may be facilitated by the electrowetting arrangement. Detachment from the outlet 114 may occur when the sweat droplet has grown to acquire a sufficiently large diameter that the sweat droplet at least partially overlaps a pair of electrodes 116. In this case, once an electrowetting wave passes along the electrodes 116, the sweat droplet spanning the pair of electrodes 116 may be dislodged from the outlet 114 accordingly. In such an example, the sweat droplets may not be all of a uniform size or volume, because the sweat droplet may continue to grow to varying degrees in the period between the sweat droplet reaching the requisite diameter and the arrival of the electrowetting wave. In this respect, the sweat droplet size may be determined by the frequency of the electrowetting wave.

Accordingly, arranging the electrodes 116 in the further substrate 118 should not be regarded as being limiting, and in alternative non-limiting examples the electrodes 116 of the electrowetting arrangement may be arranged in the substrate 110. In the latter case, the sweat droplets may be released and transported along a hydrophobic layer 118A defining the upper surface of the substrate 110.

More generally, the released sweat droplet may be transported at least as fast as the subsequent sweat droplet protrudes from the outlet 114. Preferably, migration of a sweat droplet is faster than formation, i.e. the protruding, of the subsequent sweat droplet. This is in order to ensure unambiguous sweat droplet definition, i.e. to ensure transport of a train of discrete sweat droplet.

In other words, the fluid transport assembly may maintain the discrete droplet characteristics of the sweat droplets by ensuring rapid transport/migration relative to sweat droplet formation. This may have advantages over a continuous flow of sweat, especially at low sweat rates, in terms of lessening or avoiding diffusion of components, such as biomarkers, between sweat samples collected at different points in time.

It is also noted that, whilst not visible in the cross-sectional representation provided in Fig. 1, the passages along which the sweat droplets are transported may be at least partially, and preferably fully, enclosed in order to minimize evaporation of the sweat droplets during their transportation by the electrowetting arrangement.

In the non-limiting example shown in Fig. 1, also shown in Fig. 2, the electrowetting arrangement comprises at least one series 124 of electrodes 116, with each electrode 116 being configured to release and/or transport one of the sweat droplets protruding from a respective outlet 114. Moreover, in this example, the electrowetting arrangement further comprises at least one further series 126 of electrodes 116 configured to receive sweat droplets from the, e.g. each, series 124 of electrodes 116, and transport said sweat droplets away from the at least one series 124 of electrodes 116.

The sweat droplet pathway defined by the series 124 of electrodes 116 may be regarded as a "level zero track", and the sweat droplet pathway defined by the further series 126 of electrodes 116 may be regarded as a "level one track".

In the non-limiting example shown in Fig. 1, five electrodes 116 of the series 124 are associated with six cylindrical chambers 102, although alternative chamber 102 numbers (and shapes) may be contemplated, such as two, three, four, five, seven, eight or more.

There may be no chambers 102, and hence no outlets 114, associated with, e.g. opposing, the electrodes 116 of the further series 126. Thus, the electrodes 116 of the further series 126 may, in such an example, be solely employed for transporting sweat droplets, e.g. to or towards at least one sensor (not visible in Fig. 1).

Fig. 2 shows a plan view of part of the substrate 110 which shows ten series 124A-J, in other words ten level zero tracks, and one further series 126, in other words one level one track. Also evident in Fig. 2 are the outlets 114 of each of the chambers 102.

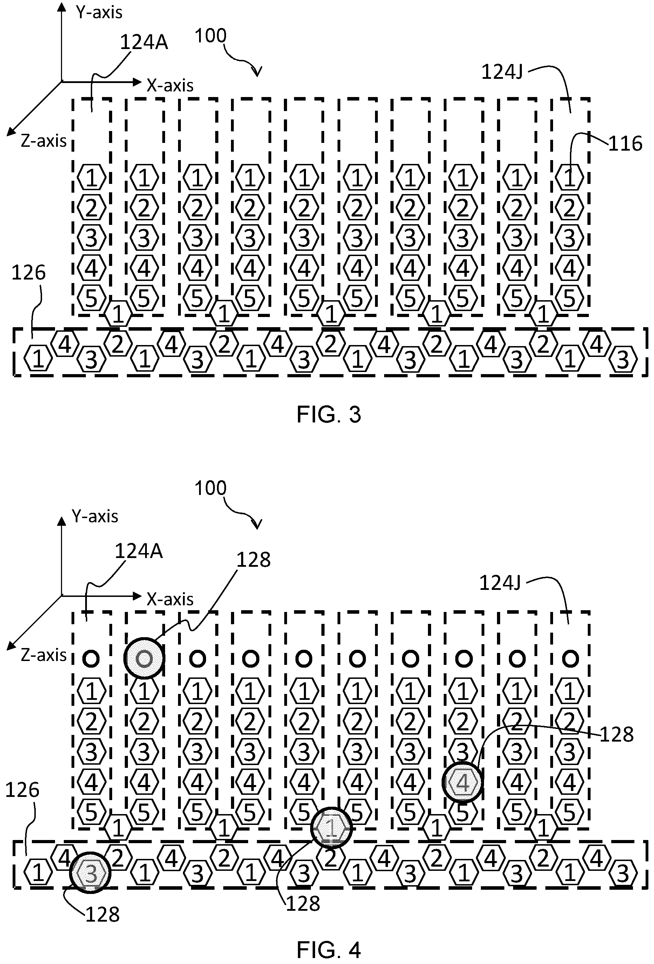

Fig. 3 shows a part of the further substrate 118, in particular showing the electrodes 116 of the electrowetting arrangement. In this particular example, hexagonally shaped electrodes 116 are employed. In this manner, the density of chambers 102/outlets 114 may be increased. However, any suitable shape for the electrodes, when view in plan, may be contemplated, such as square or rectangular. Alternatively or additionally, the periphery, e.g. circumference, of these electrodes may have regular protrusions and indents to improve movement of a droplet. Droplets may always have to be in contact with the surface above the subsequent electrode to induce movement.

Fig. 4 shows sweat droplets 128 being transported using the apparatus 100 shown in Fig. 3. An electrowetting wave is induced by charging/discharging the electrodes 116 numbered 1 to 5 in the level zero tracks 124A-J. The second electrowetting wave in the level one track 126 is induced by sequentially charging/discharging the electrodes 116 numbered 1 to 4.

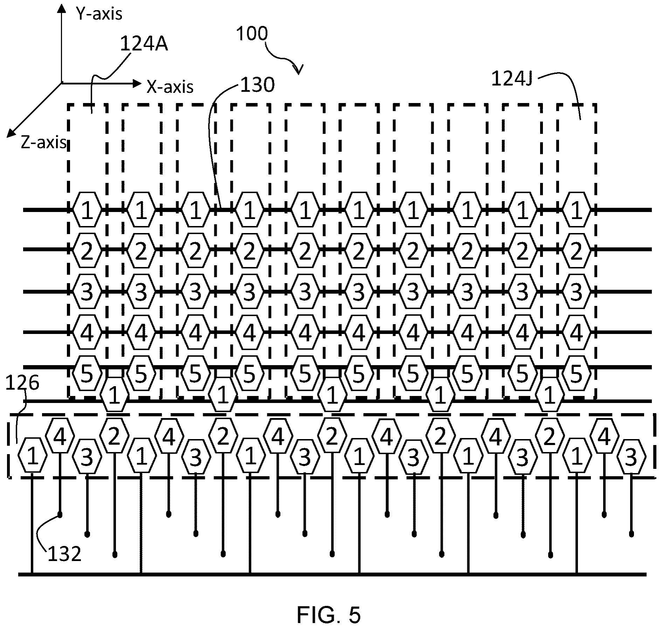

Fig. 5 shows an example, where conductive traces 130 connect the electrodes 116 shown in Figs. 3 and 4. Each of the electrodes 116 numbered 1 in the level zero tracks 124A-J are connected together to define a set of electrodes 116. The same is true of the electrodes 116 numbered 2, the electrodes 116 numbered 3, the electrodes 116 numbered 4, and the electrodes 116 numbered 5. Thus, the electrodes 116 of the level zero tracks only require six connections: five connections to charge/discharge the electrodes 116 and one connection (not visible) that connects to the mutual shared earth or neutral (see the ground electrode 120 described above in relation to Fig. 1). Nevertheless, it is to be understood that more or less than five electrodes 116 may be used in the level zero tracks. If a number of N electrodes are used, then the number of the needed connections will be N + 1, where the extra 1 connection is the mutual shared earth or neutral.

By the electrodes 116 of the level zero tracks 124A-J being connected to each other in sets (numbered 1 to 5) in this way, the complexity of the electrical connection scheme connecting each electrode 116 to an electric field generator, e.g. an electronic control board of such an electric field generator, may be significantly reduced relative to the scenario in which each electrode 116 is connected to the electric field generator individually.

In the example shown in Fig. 5, the level one track 126 also utilizes sets of electrodes 116, in this case sets of electrodes 116 numbered 1 to 4, to transport sweat droplets. To enable electrowetting waves over the level one track 126, fifteen VIAs 132 are required for an apparatus 100 having ten level zero tracks 124A-J, as shown.

It is noted that any suitable number of electrodes 116 can be used in each of the level zero track(s) 124A-J, for example two, three, four, five, six, seven, eight or more.

In some embodiments, the level one track 126 transports sweat droplets 128 towards and through a sweat rate sensor (not visible in Fig. 5), which sweat rate sensor is configured to count the number of sweat droplets 128 passing therethrough and also the time take for each sweat droplet 128 to pass through the sweat rate sensor.

Moreover, in some embodiments a biosensor (not visible in Fig. 5) may be arranged downstream of such a sweat rate sensor. When a sweat droplet 128 which has passed through the sweat rate sensor is further transported by the level one track 126 to the biosensor a concentration of a biomarker in the sweat can be determined. Other sensors suitable to receive a sweat sample, may also be used.

Since each sequence of electrodes (numbered 1 to 4) of the level one track 126 is followed by a subsequent sequence, each sweat droplet 128 can be seamlessly transported along the level one track 126 without interruption. Defining an electrowetting wave as sequential charging/de-charging of the sets of electrodes 116, in the example shown in Fig. 5, ten of these waves are conducted simultaneously on the level zero tracks 124A-J but also 5 waves are conducted in the level one track 126. This way, a sweat droplet 128, as shown in Fig. 4, may experience seamless transport first over a level zero track 124A-J and subsequently over the level one track 126, and, as such from the standpoint of that sweat droplet 128 it can be regarded as experiencing one large electrowetting wave towards the sweat rate sensor and/or the biosensor.

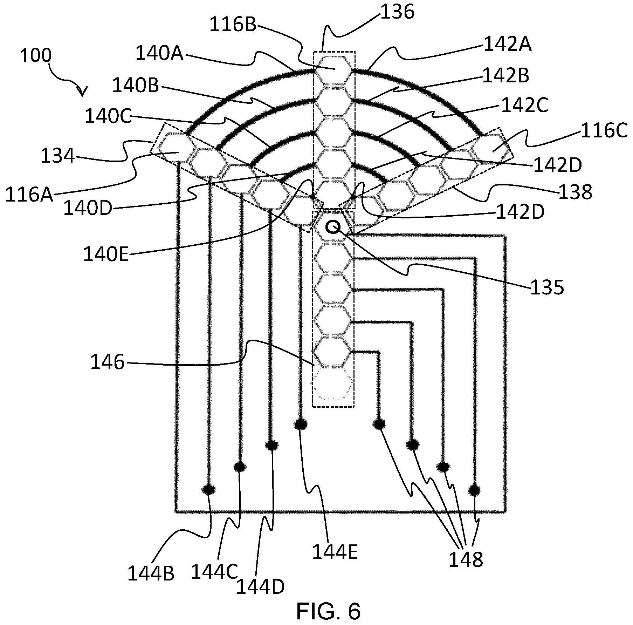

More generally, and referring to Fig. 6, the outlets 114 are radially grouped around a central portion 135 of the surface of the substrate 110 to define at least first, second and third groups of chambers 102. Moreover, the electrowetting arrangement comprises a first limb 134, a second limb 136, and a third limb 138 of electrodes 116 configured to permit transport of the sweat droplets from the first group in the case of the first limb 134 via an electrode located at the central portion 135, in the same manner centrally from the second group in the case of the second limb 136, and in the same manner centrally from the third group in the case of the third limb 138 by charging and discharging of the electrodes 116. The groups of chambers 102 are not shown in Fig. 6, as they are located under the limbs, i.e. in the example shown in Fig. 6, the first group of chambers 102 is located under the first limb 134, the second group of chambers 102 is located under the second limb 136 and the third group of chambers is located under the third limb 138.

Fig. 6 provides a plan view of an exemplary apparatus 100 in which the first limb 134 comprises, or more specifically is defined by, a series of first electrodes 116A, each first electrode 116A of the series being configured to release and/or transport one of the sweat droplets protruding from a respective outlet in the first group of chambers 102. The first limb 134/series of first electrodes 116A in this example can thus be regarded as a level zero track.

Similarly, the second limb 136 comprises, or more specifically is defined by, a series of second electrodes 116B, each second electrode 116B of the series being configured to release and/or transport one of the sweat droplets protruding from a respective outlet in the second group of chambers 102. The second limb 136/series of second electrodes 116B in this example can thus be regarded as a level zero track.

With continued reference to Fig. 6, the third limb 138 comprises, or more specifically is defined by, a series of third electrodes 116C, each third electrode 116C of the series being configured to release and/or transport one of the sweat droplets protruding from a respective outlet in the third group of chambers 102. The third limb 138/series of third electrodes 116C in this example can thus be regarded as a level zero track.

By the first, second and third limbs 134, 136, 138 transporting the sweat droplets 128 centrally, e.g. generally radially, from the first, second and third groups respectively, the apparatus 100 may be particularly suitable for incorporation in, for instance, a wearable sweat sensing device, e.g. in the form of a wearable patch.

In such a wearable sweat sensing device, one or more sensors, such as the above-described sweat rate sensor and/or biosensor, are preferably arranged in a central region of the apparatus 100 to or towards which the sweat droplets are transported via the first, second and third limbs 134, 136, 138. The arrangement of the limbs 134, 136, 138, which in at least some embodiments can be regarded as a radial configuration of limbs 134, 136, 138, may assist to minimize the transportation distance from the outlet(s) positioned farthest from the central region to the sensor(s). This may, in turn, assist to minimize evaporation of sweat droplets 128 during their transportation to the sensor(s).

The electrowetting arrangement also comprises a conductive trace arrangement arranged to electrically connect electrodes 116 of the first, second, and third limbs 134, 136, 138 to each other. By the conductive trace arrangement being arranged to electrically connect electrodes of the first, second, and third limbs 134, 136, 138 to each other, the physical design of the apparatus 100 may be simplified and/or made more robust, e.g. since fewer vertical interconnect access (VIA) connections may be required and/or the use of matrix driver layer to control the charging and discharging of the electrodes 116 may be obviated.

As shown in relation to the non-limiting example shown in Fig. 6, the plurality of electrodes 116 comprises a plurality of sets of electrodes, each set comprising electrodes 116A, 116B, 116C from the first, second and third limbs 134, 136, 138 which are connected to each other.

A first conductive trace 140A; 140B; 140C; 140D; 140E of the conductive trace arrangement is configured to connect one of the electrodes 116A, particularly one of the series of first electrodes 116A in the case of the example shown in Fig. 6, of the first limb 134 to one of the electrodes 116B, particularly one of the series of second electrodes 116B in this case, of the second limb 136. Moreover, a second conductive trace 142A; 142B; 142C; 142D; 142E of the conductive trace arrangement is configured to connect said one of the electrodes 116B of the second limb 136 (connected to one of the electrodes 116A of the first limb 134) to one of the electrodes 116C, particularly one of the series of third electrodes 116C, of the third limb 138.

The outlets 114 and affiliated electrodes 116A, 116B, 116C of the level zero tracks 134, 136, 138 shown in Fig. 6 can be regarded as being arranged in an approximately half circular design (also termed a "cauliflower design" herein). This may provide a relative compact apparatus 100.

In the non-limiting example shown in Fig. 6, the electrodes 116A, 116B, 116C of all the level zero tracks, corresponding to the first, second and third limbs 134, 136, 138, are connected to five electrical lines and only four VIAs 144B, 144C, 144D, 144E are required to provide an electrical connection that enables the abovementioned electrowetting wave to propagate on the level zero tracks 134, 136, 138.

In the non-limiting example shown in Fig. 6, a sensor transport limb 146 transports sweat droplets from the first, second and third limbs 134, 136, 138 downstream towards one or more sensors (not visible in Fig. 6), such as a sweat rate sensor and/or a biosensor. In this case, the sensor transport limb 146 corresponds to a level one track whose electrodes 116 are connected by VIAs 148.

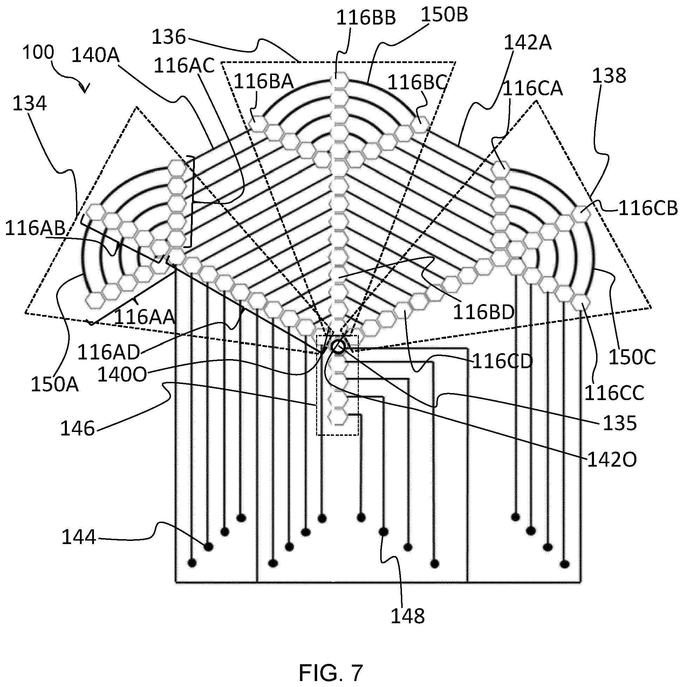

In some embodiments, such as in the non-limiting example shown in Fig. 7, the first limb 134 comprises at least one series of first electrodes 116AA, 116AB, 116AC, and at least one further series of first electrodes 116AD configured to receive sweat droplets from each series of the at least one series of first electrodes 116AA, 116AB, 116AC, and transport said sweat droplets away, in this case centrally away, from the at least one series of first electrodes 116AA, 116AB, 116AC.

Each of the at least one series of first electrodes 116AA, 116AB, 116AC constitutes a level zero track, and each of the at least one further series of first electrodes 116AD constitutes a level one track.

The second limb 136 may also comprise at least one series of second electrodes 116BA, 116BB, 116BC, and at least one further series of second electrodes 116BD configured to receive sweat droplets from each series of the at least one series of second electrodes 116BA, 116BB, 116BC, and transport said sweat droplets away, in this case centrally away, from the at least one series of second electrodes 116BA, 116BB, 116BC.

Each of the at least one series of second electrodes 116BA, 116BB, 116BC constitutes a level zero track, and each of the at least one further series of second electrodes 116BD constitutes a level one track.

Similarly, the third limb 138 may comprise at least one series of third electrodes 116CA, 116CB, 116CC, and at least one further series of third electrodes 116CD configured to receive sweat droplets from each series of the at least one series of third electrodes 116CA, 116CB, 116CC, and transport said sweat droplets away, in this case centrally away, from the at least one series of third electrodes 116CA, 116CB, 116CC.

Each of the at least one series of third electrodes 116CA, 116CB, 116CC constitutes a level zero track, and each of the at least one further series of third electrodes 116CD constitutes a level one track.

Similarly to the example shown in Fig. 6, the plurality of electrodes comprises a plurality of sets of electrodes in the electrowetting arrangement depicted in Fig. 7, with each set comprising electrodes 116AB-AD; 116BA-BD, 116CA-CD from the first, second and third limbs 134, 136, 138 which are connected to each other.

A first conductive trace 140A-O of the conductive trace arrangement is configured to connect one of the electrodes of the first limb 134 to one of the electrodes of the second limb 136. Moreover, a second conductive trace 142A-O of the conductive trace arrangement is configured to connect said one of the electrodes of the second limb 136 (connected to one of the electrodes of the first limb 134) to one of the electrodes of the third limb 138.

Moreover, as shown in relation to the non-limiting example shown in Fig. 7, at least one further conductive trace 150A, 150B, 150C is configured to connect electrodes within one of the limbs 134, 136, 138 to each other.

In the example shown in Fig. 7, each of the first, second and third limbs 134, 136, 138 comprises three level zero tracks, with two further conductive traces 150A, 150B, 150C per limb connecting the level zero tracks within each limb to each other, as shown.

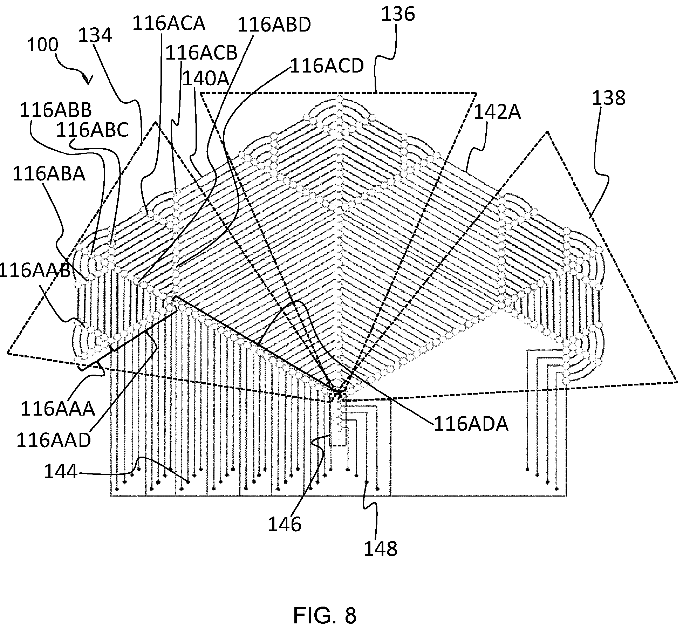

In some embodiments, such as in the non-limiting example shown in Fig. 8, the first limb 134 comprises a plurality of series of first electrodes 116AAA, 116AAB, 116ABA, 116ABB, 116ABC, 116ACA, 116ACB, a plurality of further series of first electrodes 116AAD, 116ABD, 116ACD, and an additional series of first electrodes 116ADA configured to receive sweat droplets from each further series of the plurality of further series of first electrodes 116AAD, 116ABD, 116ACD, and transport said sweat droplets away, in this case centrally away, from the plurality of further series of first electrodes 116AAD, 116ABD, 116ACD.

The additional series of first electrodes 116ADA can be regarded as a level two track.

In the non-limiting example shown in Fig. 8, the second and third limbs 136, 138 also each comprise a plurality of series of first electrodes, a plurality of further series of first electrodes, and an additional series of first electrodes.

Similarly to the examples shown in Fig. 7, the plurality of electrodes comprises a plurality of sets of electrodes in the apparatus depicted in Fig. 8, and a first conductive trace 140A of the conductive trace arrangement is configured to connect one of the electrodes of the first limb 134 to one of the electrodes of the second limb 136. Moreover, a second conductive trace 142A is configured to connect said one of the electrodes of the second limb 136 (connected to one of the electrodes of the first limb 134) to one of the electrodes of the third limb 138. Moreover, further conductive traces are included in the conductive trace arrangement, with each further conductive trace being configured to connect electrodes within one of the limbs 134, 136, 138 to each other.

The number of outlets 114 and VIAs 144, 148 in the exemplary apparatuses 100 shown in Figs. 6-8 are tabulated in Table 1, together with a further example (not depicted) having level one to level three tracks. Table 1 shows that, in general, an increasing number of outlets 114, in other words chambers 102, may require a higher number of VIAs.

**Table 1.**

| Design | Number of outlets 114 | Number of VIAs 144, 148 |
|---|---|---|
| Fig. 6 | 18 | 8 |
| Fig. 7 | 54 | 16 |
| Fig. 8 | 132 | 36 |
| Design with level one to level three tracks (not visible) | 288 | 84 |

Many more outlets 114 may be required in certain cases. For instance, if each outlet 114 is 30 µm in diameter and the exit of the gland duct 108 onto the skin 106 is about 40 µm, the skin surface area that is sampled by one outlet 114 has a diameter of about 113 µm (providing at least partial overlap with the exit of a gland 108). Typically, in sedentary state about 10 eccrine glands are active per cm² skin. To provide sufficient reliability one would like to measure about 10 glands. So, a cm² of skin may be sampled. This would require about 10000 outlets 114. Note that with higher sweat production the number of active sweat glands per surface area can increase (typically a factor 2 to 10) but still a large amount of collection chambers may be required and hence the number of required VIAs is still significant. When a cauliflower design is provided for serving 10000 outlets 114, without using the teachings of this invention, the number of VIAs may be in the order of thousands. An extrapolation has shown that 4296 VIAs may be required.

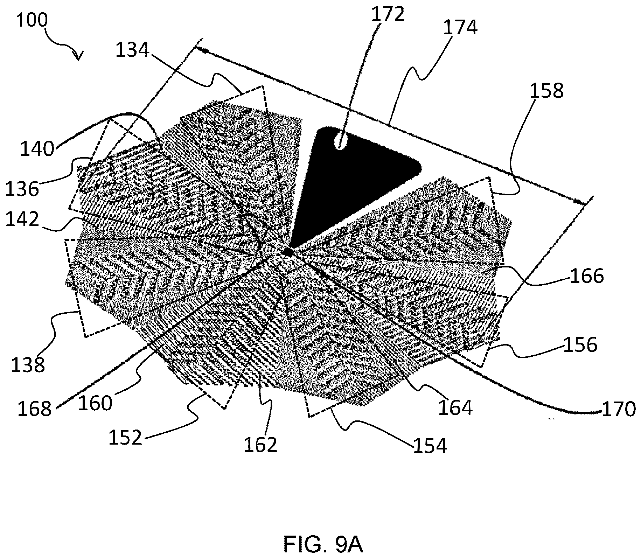

Fig. 9A provides a perspective view of an apparatus 100 according to another non-limiting example. In this case, the electrowetting arrangement comprises seven limbs 134, 136, 138, 152, 154, 156, 158. More generally, any suitable number of limbs 134, 136, 138, 152, 154, 156, 158 beyond three can be contemplated, such as four, five, six, seven, eight, nine, ten, eleven, twelve or more.

In this example, the plurality of electrodes comprises a plurality of sets of electrodes, with each set comprising electrodes from the first, second, third, fourth, fifth, sixth, and seventh limbs 134, 136, 138, 152, 154, 156, 158. A first conductive trace 140 is configured to connect one of the electrodes of the first limb 134 to one of the electrodes of the second limb 136, a second conductive trace 142 is configured to connect said one of the electrodes of the second limb 136 to one of the electrodes of the third limb 138, a third conductive trace 160 is configured to connect one of the electrodes of the third limb 138 to one of the electrodes of the fourth limb 152, a fourth conductive trace 162 is configured to connect one of the electrodes of the fourth limb 152 to one of the electrodes of the fifth limb 154, a fifth conductive trace 164 is configured to connect one of the electrodes of the fifth limb 154 to one of the electrodes of the sixth limb 156, and a sixth conductive trace 166 is configured to connect one of the electrodes of the sixth limb 156 to one of the electrodes of the seventh limb 158.

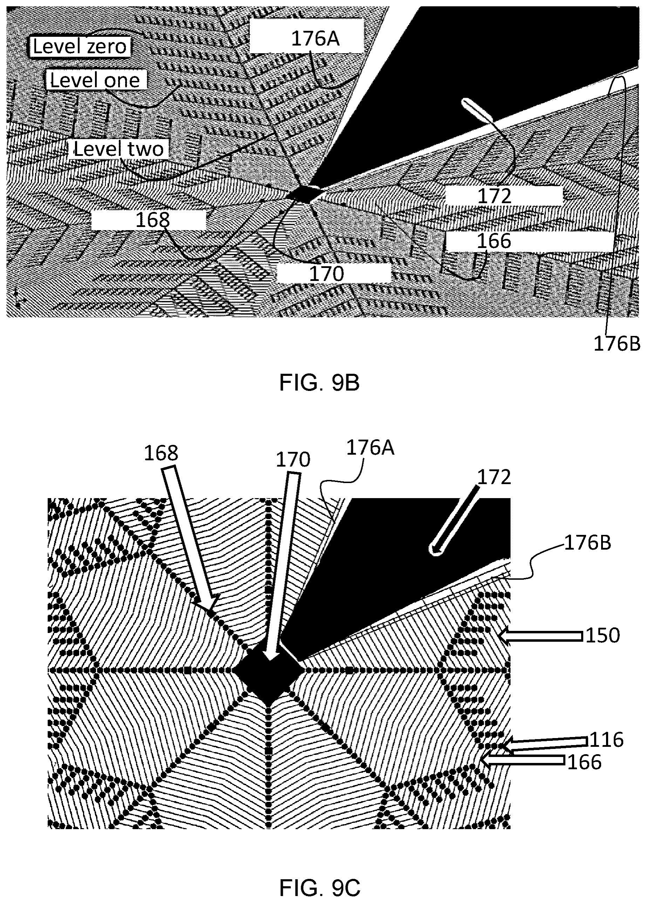

Moreover, similarly to conductive traces 150A, 150B, 150C shown in Fig. 7, at least one further conductive trace included in the conductive trace arrangement (see reference numeral 150 in Fig. 9C) is configured to connect electrodes within one of the limbs 134, 136, 138, 152, 154, 156, 158 to each other.

In some embodiments, including the non-limiting example shown in Fig. 9A and in the enlargement of Fig. 9A in Fig. 9B, a first sweat rate sensor, a second sweat rate sensor, and/or a third sweat rate sensor 168 is included in the apparatus 100. In such embodiments, the first sweat rate sensor is arranged downstream of the outlets 114 of the first group and configured to determine a sweat rate from sweat droplets transported by the first limb 134, the second sweat rate sensor is arranged downstream of the outlets 114 of the second group and configured to determine a sweat rate from sweat droplets transported by the second limb 136, and the third sweat rate sensor 168 is arranged downstream of the outlets 114 of the third group and configured to determine a sweat rate from sweat droplets transported by the third limb 138.

A single sweat rate sensor 168 may be used in the apparatus 100, but preferably, a sweat rate sensor 168 is provided per limb 134, 136, 138. This may assist the apparatus 100 to determine a sweat rate per sweat gland. In the non-limiting example shown in Fig. 9A, seven sweat rate sensors 168 are provided, one for each of the seven limbs 134, 136, 138, 152, 154, 156, 158.

In the example shown in Fig. 9A, the electrowetting arrangement comprises level zero, level one, and level two tracks, similarly to the example depicted in Fig. 8. A sweat rate sensor 168 is provided on each of the level two tracks, as shown.

The sweat rate sensor 168 may be configured to count the sweat droplets transported thereto, and in at some cases also determine the volume of each of the sweat droplets.

Any suitable sensing principle can be employed for the sweat rate sensor 168. For example, a capacitance sensing principle may be particularly useful for counting the sweat droplets. Such a sensing principle may also enable estimation of the time during which the sweat droplet passes through the detector, i.e. between the plates of the capacitor, since the dielectric change between air and a sweat droplet (about 99% water) is relatively large (about a factor of 80). The time taken for the sweat droplet to pass through the sweat rate sensor may be indicative of the volume of the sweat droplet.

Alternatively or additionally, the apparatus 100 may comprise a biosensor 170 configured to determine a sweat analyte concentration. The biosensor 170 may be arranged to receive sweat droplets transported thereto by each of the limbs 134, 136, 138, 152, 154, 156, 158.

In at least some embodiments, the apparatus 100 further comprises a sweat collector 172 downstream of the biosensor 170. The sweat collector 172 may provide a storage reservoir for sweat which has been analyzed by the biosensor 170. For example, the sweat collector 172 can be in the form of an absorber/absorbent pad, in other words an absorbent material, configured to absorb the sweat transported thereto.

The apparatus 100 shown in Fig. 9A may have about 10000 outlets 114. For reference, the diameter 174 of the apparatus 100 in this example is 27.35 mm.

In the example shown in Fig. 9A, sweat droplets are transported along the seven feather-like limbs 134, 136, 138, 152, 154, 156, 158, with each limb comprising level zero tracks, level one tracks, and a level two track. Droplets may move from a level zero track to a level one track and subsequently to a level two track which also defines a path through the sweat rate sensor 168 where the droplets are counted and further transported to the biosensor 170, where a biomarker concentration is measured. Finally, the liquid of the droplets is collected in the sweat collector 172, in this case absorber.

Further details can be observed from the enlarged views provided in Figs. 9B-E. As best shown in Figs. 9B and 9C, each of the conductive traces of the conductive trace arrangement extend from proximal to a first radially extending edge 176A, e.g. of the further substrate 118, around to proximal a second centrally extending edge 176B, e.g. of the further substrate 118. The VIAs may, for example, be provided proximal to the first and/or second radially extending edges 176A, 176B.

In the depicted example, the sweat collector 172 is arranged between the first and second radially extending edges 176A, 176B.

As best shown in Fig. 9C, one electrical path connects several electrodes, but with all of such electrodes having the same number, in this case numbered "1", "2", "3", "4", and "5" - see also Figs. 3-5. In this example, the five electrical paths in series form a grouping, and such groupings are repeated starting from about the periphery of the biosensor 170 radially outwards.

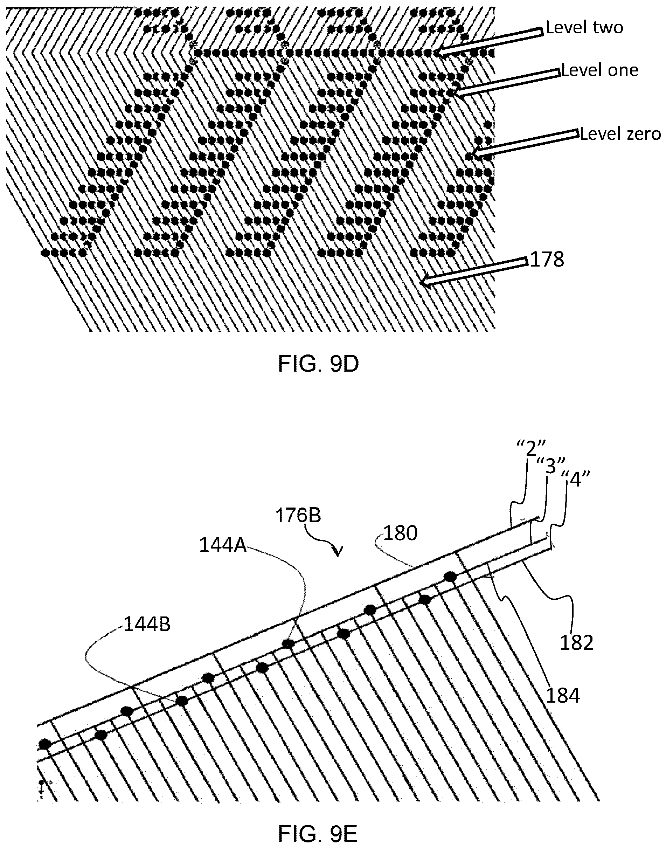

The level zero, level one, and level two tracks and the conductive traces are clearly observable in the enlarged view provided in Fig. 9D. In the upper part of Fig. 9D, a change in direction of the conductive traces can be seen. This point of direction change is also a mirror plane for the electrodes of the same limb. As such, the electrical traces start proximal to one of the radially extending edges 176A, 176B, and end proximal to the other of the radially extending edges 176B, 176A via a plurality of such direction changes.

At this point it is noted that in the embodiments depicted in Figs. 6-8, the level zero tracks all transport sweat droplets to a first point being the start of a level one track. Subsequently, these level one tracks move sweat droplets to a second point being the start of a level two track. Finally, these level two tracks transport droplet to a third point, being the start of one level three track transporting droplets to the central sensor area.

The embodiment of Figs. 9A-D is a preferred modification of this theme: the level zero tracks move droplets to a level one track in linear fashion (so not to one first central point, but exiting onto several locations of the level one track), subsequently these level one tracks exit onto a level two track again in a linear fashion (again not to one central point, but exiting on several locations of the level two track) and finally the level two tracks transport droplets over each a sweat rate sensor and towards a central point which overlays with the biosensor.

In this way, the average density of the chambers 102 may be larger, e.g. than with the design shown in Figs. 6-8. The latter can only use the periphery of the device to place the chambers 102. By optimizing the density of the chambers in the manner of the embodiment shown in Figs. 9A-D, a smaller patch can be placed on the skin with the same performance.

Fig. 9D shows conductive traces 178 extending at an angle with respect to the direction of extension of the level zero, level one and level two tracks.

In this particular example, there are four electrodes 116 and so five outlets 114 affiliated to one level zero track. Also, another number of electrodes can be chosen, such as 5 or 6 or 7 electrodes per level zero track. Further, there are twelve level zero tracks affiliated to one level one track, although the number of level zero tracks per level one track may be less towards the centre of the electrowetting arrangement due to space limitations. In this example, each of the 11th level one tracks has nine level zero tracks and the last being the 12th level one tracks has six level zero tracks. Subsequently these twelve level one tracks connect to one level two track, and due to mirroring in total twenty four level one tracks are connected to one level two track (constituting one of the feather-like limbs 134, 136, 138, 152, 154, 156, 158).

There are thirty electrical paths to connect the most peripheral level zero tracks. In addition, there are 220 electrical paths to also connect all level one tracks to the level two tracks and finally about 25 electrical paths connect the level two tracks up to the biosensor 170. Consequently, there are 275 electrical paths starting proximal to one of the radially extending edges 176A, 176B and ending at the other of the radially extending edges 176B, 176A. This constitutes 55 groupings of five electrodes.

Proximal to one of the radially extending edges 176A, 176B there are 2 VIAs per grouping and proximal to the other radially extending edge 176B, 176A there is one VIA of this grouping. So proximal to the first radially extending edge 176A there are in total 110 VIAs and proximal to the other radially extending edge there are in total 55 VIAs. This gives a total of 155 VIAs.

Further, it can be calculated that twelve level one tracks including the affiliated level zero tracks can serve 675 outlets 114, and that one level two track, with affiliated level one and level zero tracks, can serve 1350 outlets 114. Since there are seven level-two tracks, the whole electrowetting arrangement serves 9450 outlets 114. Just for a comparison, scaling linearly to 10000 outlets 114 there are only about 175 VIAs required.

For comparison, individually addressed electrodes 116 of an electrowetting arrangement serving 10000 outlets would require 10000 VIAs. Thus, the design shown in Figs. 9A-D can provide a reduction in the number of VIAs by around a factor of sixty.

Fig. 9E provides an enlarged view of one of the radially extending edges 176B, which also shows the VIAs 144A for connecting all of the electrodes numbered "3", and the VIAs 144B for connecting all the electrodes numbered "4". No VIA is required for connecting the electrodes numbered "2".

Electrical pathways for the electrodes numbered "1" and "4" are located proximal to the other radially extending edge 176A (not visible in Fig. 9E).

The VIAs 144B connect to an additional conductive trace 182 provided on a surface opposing the surface on which the electrical traces connecting the electrodes are arranged. Similarly, the VIAs 144A connect to another additional conductive trace 184 provided on the surface opposing the surface on which the electrical traces connecting the electrodes are arranged.

What remains is to calculate the transport time of a sweat droplet originating from the outlets 114 the furthest away from the biosensor 170 for the example depicted in Figs.9A-E. The number of electrodes 116 is: four electrodes of the furthest away level zero track; 25 electrodes of the affiliated level one track; and 245 electrodes of the affiliated level two track. In total, there are 274 electrodes, hence 275 steps from the farthest outlet 114 to the biosensor 170.

A typical step time of the electrowetting wave may be 0.1 to 1 second. The electrowetting wave may be defined as charging a first electrode, keeping the charge for a certain time, de-charging this electrode and during de-charging start this cycle on the adjacent subsequent electrode and so on, as previously described.

Assuming a step time of 0.1 second, the longest total transport time is 27.5 seconds for the non-limiting example shown in Figs. 9A-E. This relatively short longest total transport time may advantageously minimize evaporation of sweat droplets during transportation to the sensor(s) 168, 170.

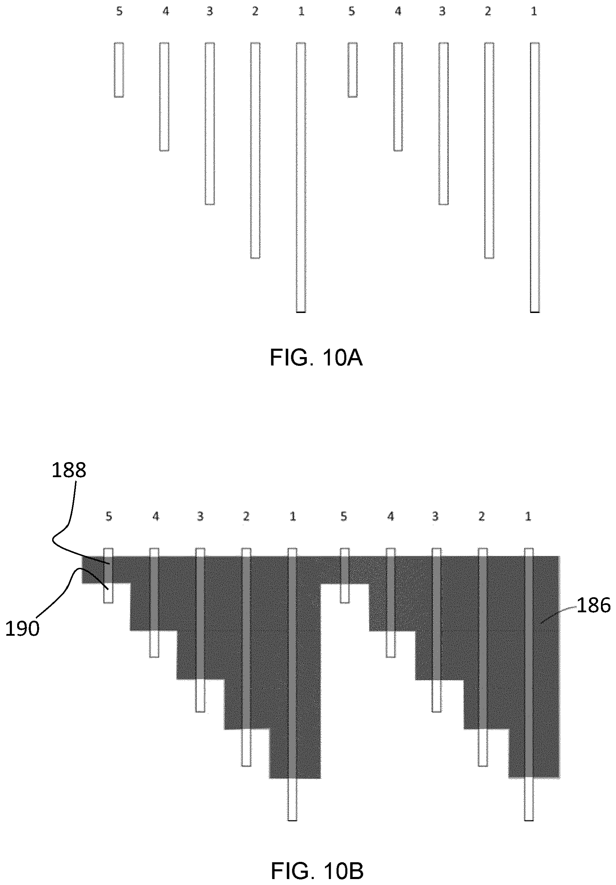

Whilst Fig. 9E shows VIAs 144A, 144B arranged proximal to one of the radially extending edges 176B, in some embodiments such VIAs can be omitted. Figs. 10A-D schematically depict a manufacturing process which can be used as an alternative to forming such VIAs.

Fig. 10A schematically depicts a first step in the process providing the conductive trace arrangement. Such a first step may, for example, include an initial deposition of chromium followed by a subsequent deposition of gold on a substrate, e.g. the further substrate 118. A glass (further) substrate 118 may, for instance, be used for this purpose.

In Fig. 10A only the ends of the conductive trace arrangement are shown, and in particular the ends of two groupings of electrodes numbered 1 to 5.

The ends of the conductive trace for the electrodes numbered 1 terminate furthest away from where the ends of the conductive trace for the other electrodes, followed by the electrodes numbered 2, and so on.

The ends of the conductive trace arrangement may, in the case of the example depicted in Figs. 9A-E, be proximal to one of the radially extending edges 176A, 176B.

In the step shown in Fig. 10B, an electrical isolator layer 186 is deposited onto the conductive trace arrangement, and in this particular example onto the gold layer of the conductive trace arrangement, such that an isolated portion 188 of each conductive trace is covered with the isolator layer 186, and an exposed portion 190 is not covered by the isolator layer 186.

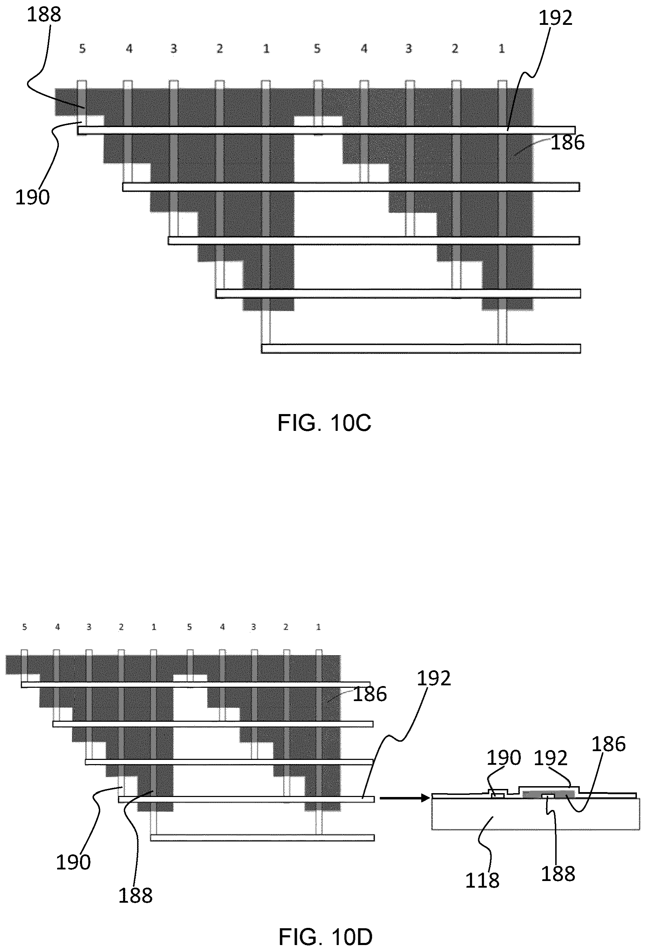

In the step shown in Fig. 10C, a further conductive layer 192, e.g. comprising chromium and then gold, is deposited, mainly on the isolator layer 186. However, a conductive trace of the further conductive layer 192 extends between the exposed portions 190 of the conductive traces connecting the electrodes numbered 1, but is isolated by the isolator layer 186 from all of the other conductive traces (numbered 2, 3, 4, and 5). The ends of the conductive traces connecting electrodes numbered 2, 3, 4, and 5 are analogously connected.

Thus, and as best shown in Fig. 10D, the isolator layer 186 prevents an electrical short circuit of the electrical paths such that zero VIAs may be required.

The isolator layer 186 could be for instance a ceramic layer or a polymer i.e. Parylene C. In the case the edge thickness of the isolator is decreasing too gradually, a state-of-the-art photolithographic process can be utilized to remove a part of the isolator layer, rendering an edge of the isolator with a more stepwise fashion in thickness. In short, an additional protective layer is applied that is subsequently illuminated through a photolithographic mask. Subsequently certain areas of this protective layer are washed away creating a pattern. Subsequently, an etching step is applied which removes the isolator layer in the appropriate locations, there were the protection layer was removed. Subsequently the protective layer is removed.

The apparatus, systems and methods of the present disclosure may be applied for non-invasive, semi-continuous and prolonged monitoring of biomarkers that indicate health and well-being, for example for monitoring dehydration, stress, sleep, children's health and in perioperative monitoring. As well as being applicable for subject monitoring in general, the present apparatus, systems and methods may be specifically applied to provide an early warning for sudden deterioration of patients in the General Ward and Intensive Care Unit, or for investigation of sleep disorders. Currently, measurements may only be made in a spot-check fashion when a patient is visiting a doctor, although it is noted that the present disclosure may also be usefully applied in performing such spot-check measurements.

## Claims

1. An apparatus (100) for transporting sweat droplets, the apparatus comprising:
a plurality of chambers (102) defined in a substrate (110), each of the chambers having an inlet (104) for receiving sweat from the skin, and an outlet (114) arranged such that a sweat droplet forms and protrudes therefrom following filling of the chamber with sweat;
wherein the outlets are radially grouped around a central portion (135) of the surface of the substrate to define at least first, second and third groups of chambers; and
an electrowetting arrangement comprising:
a plurality of electrodes (116), each electrode being electrically chargeable and dischargeable, wherein the electrowetting arrangement comprises first, second, and third limbs (134, 136, 138) of electrodes configured to permit transport of the sweat droplets to or towards a central region from the first group in the case of the first limb, to or towards the central region from the second group in the case of the second limb, and to or towards the central region from the third group in the case of the third limb by charging and discharging of the electrodes; and
a conductive trace arrangement (140A, 140B, 140C; 142A, 142B, 142C; 150A, 150B, 150C) arranged to electrically connect electrodes of the first, second, and third limbs to each other,
**characterized in that**
the plurality of electrodes (116) comprises a plurality of sets of electrodes, each set comprising electrodes from the first, second and third limbs (134, 136, 138) which are connected to each other, **further characterized in that,** for each set, a first conductive trace (140A; 140B; 140C) included in the conductive trace arrangement is configured to connect one of the electrodes of the first limb (134) to a corresponding electrode of the second limb (136), and a second conductive trace (142A; 142B; 142C) included in the conductive trace arrangement is configured to connect said corresponding electrode of the second limb (136) to a corresponding electrode of the third limb (138), and
**further characterized in that,** for each set, at least one further conductive trace (150A, 150B, 150C) is included in the conductive trace arrangement and configured to connect electrodes within one of the first, second, and third limbs (134, 136, 138) to each other.

2. The apparatus (100) according to claim 1, wherein the first limb (134) comprises at least one series of first electrodes (116A), each electrode of the at least one series of first electrodes being configured to release and/or transport one of said sweat droplets protruding from a respective outlet (114) in the first group.

3. The apparatus (100) according to claim 2, wherein the first limb (134) comprises at least one further series of first electrodes (116AD) configured to receive sweat droplets from each series of the at least one series of first electrodes (116AA, 116AB, 116AC), and transport said sweat droplets away from the at least one series of first electrodes.

4. The apparatus (100) according to claim 3, wherein the first limb (134) comprises an additional series of first electrodes (116ADA), and wherein the first limb comprises a plurality of said further series of first electrodes (116AAD, 116ABD, 116ACD), the additional series of first electrodes being configured to receive sweat droplets from each further series of the plurality of further series of first electrodes, and transport said sweat droplets away from the plurality of further series of first electrodes.

5. The apparatus (100) according to any of claims 1 to 4, wherein the second limb (136) comprises at least one series of second electrodes (116B), each electrode of the at least one series of second electrodes being configured to release and/or transport one of said sweat droplets protruding from a respective outlet (114) in the second group.

6. The apparatus (100) according to claim 5, wherein the second limb (136) comprises at least one further series of second electrodes (116BD) configured to receive sweat droplets from each series of the at least one series of second electrodes (116BA, 116BB, 116BC), and transport said sweat droplets away from the at least one series of second electrodes.

7. The apparatus (100) according to claim 6, wherein the second limb (136) comprises an additional series of second electrodes, and wherein the second limb comprises a plurality of said further series of second electrodes, the additional series of second electrodes being configured to receive sweat droplets from each further series of the plurality of further series of second electrodes, and transport said sweat droplets away from the plurality of further series of second electrodes.

8. The apparatus (100) according to any of claims 1 to 7, wherein the third limb (138) comprises at least one series of third electrodes (116C), each electrode of the at least one series of third electrodes being configured to release and/or transport one of said sweat droplets protruding from a respective outlet (114) in the third group.

9. The apparatus (100) according to claim 8, wherein the third limb (138) comprises at least one further series of third electrodes (116CD) configured to receive sweat droplets from each series of the at least one series of third electrodes (116CA, 116CB, 116CC), and transport said sweat droplets away from the at least one series of third electrodes; optionally wherein the third limb (138) comprises an additional series of third electrodes, and wherein the third limb comprises a plurality of said further series of third electrodes, the additional series of third electrodes being configured to receive sweat droplets from each further series of the plurality of further series of third electrodes, and transport said sweat droplets away from the plurality of further series of third electrodes.

10. The apparatus (100) according to claim 1, wherein:
the first limb (134) comprises at least one series of first electrodes (116A), each electrode of the at least one series of first electrodes being configured to release and/or transport one of said sweat droplets protruding from a respective outlet (114) in the first group, the first limb comprising three of said series of first electrodes,
the second limb (136) comprises at least one series of second electrodes (116B), each electrode of the at least one series of second electrodes being configured to release and/or transport one of said sweat droplets protruding from a respective outlet (114) in the second group, the second limb comprising three of said series of second electrodes,
the third limb (138) comprises at least one series of third electrodes (116C), each electrode of the at least one series of third electrodes being configured to release and/or transport one of said sweat droplets protruding from a respective outlet (114) in the third group, the third limb comprising three of said series of third electrodes, and
two further conductive traces (150A, 150B, 150C) per limb connect the three series of electrodes within each limb to each other.

11. The apparatus (100) according to any of claims 1 to 10, wherein the at least one series of first electrodes comprises a plurality of series of first electrodes, each of the plurality of series of first electrodes intersecting with one further series of said at least one further series of first electrodes at different positions relative to each other along said one further series of first electrodes; and/or wherein the at least one series of second electrodes comprises a plurality of series of second electrodes, each of the plurality of series of second electrodes intersecting with one further series of said at least one further series of second electrodes at different positions relative to each other along said one further series of second electrodes; and/or wherein the at least one series of third electrodes comprises a plurality of series of third electrodes, each of the plurality of series of third electrodes intersecting with one further series of said at least one further series of third electrodes at different positions relative to each other along said one further series of third electrodes.

12. The apparatus (100) according to any of claims 1 to 11, comprising at least one of a first sweat rate sensor, a second sweat rate sensor, and a third sweat rate sensor (168), the first sweat rate sensor being arranged downstream of the outlets (114) of the first group and configured to determine a sweat rate from sweat droplets transported by the first limb (134), the second sweat rate sensor being arranged downstream of the outlets of the second group and configured to determine a sweat rate from sweat droplets transported by the second limb (136), and the third sweat rate sensor being arranged downstream of the outlets of the third group and configured to determine a sweat rate from sweat droplets transported by the third limb (138).

13. The apparatus (100) according to any of claims 1 to 12, comprising a biosensor (170) configured to determine a sweat analyte concentration, wherein the biosensor is arranged to receive sweat droplets transported thereto by the first, second and third limbs (134, 136, 138); optionally wherein the apparatus further comprises a sweat collector downstream of the biosensor.

14. The apparatus (100) according to any of claims 1 to 13, comprising an electric field generator for charging and discharging each of the electrodes (116) of the electrowetting arrangement such as to transport each said sweat droplet.

15. A wearable device comprising the apparatus (100) according to any of claims 1 to 14; optionally wherein the wearable device comprises an attachment arrangement configured to enable attachment of the apparatus to a body part such that said inlets (104) receive sweat from the skin of said body part.

## Patentansprüche

1. Einrichtung (100) zum Transportieren von Schweißtröpfchen, wobei die Einrichtung umfasst:
eine Vielzahl von Kammern (102), die in einem Substrat (110) definiert sind, wobei jede der Kammern einen Einlass (104) zum Aufnehmen von Schweiß von der Haut und einen Auslass (114) aufweist, der derart angeordnet ist, dass sich dem Füllen der Kammer mit Schweiß folgend ein Schweißtröpfchen bildet und daraus hervortritt;
wobei die Auslässe radial um einen zentralen Abschnitt (135) der Oberfläche des Substrats herum gruppiert sind, um mindestens eine erste, eine zweite und eine dritte Gruppe von Kammern zu definieren; und
eine Elektrobenetzungsanordnung, umfassend:
eine Vielzahl von Elektroden (116), wobei jede Elektrode elektrisch aufladbar und entladbar ist, wobei die Elektrobenetzungsanordnung einen ersten, einen zweiten und einen dritten Elektrodenschenkel (134, 136, 138) umfasst, die konfiguriert sind, um im Fall des ersten Schenkels Transport der Schweißtröpfchen zu einem oder in Richtung eines zentralen Bereichs von der ersten Gruppe, im Fall des zweiten Schenkels zu dem oder in Richtung des zentralen Bereichs von der zweiten Gruppe und im Fall des dritten Schenkels zu dem oder in Richtung des zentralen Bereichs von der dritten Gruppe durch Aufladen und Entladen der Elektroden zu gestatten; und
eine Anordnung leitfähiger Spuren (140A, 140B, 140C; 142A, 142B, 142C; 150A, 150B, 150C), die angeordnet ist, um Elektroden des ersten, zweiten und dritten Schenkels elektrisch miteinander zu verbinden, **dadurch gekennzeichnet, dass** die Vielzahl von Elektroden (116) eine Vielzahl von Elektrodensätzen umfasst, wobei jeder Satz Elektroden des ersten, zweiten und dritten Schenkels (134, 136, 138) umfasst, die miteinander verbunden sind, **weiter dadurch gekennzeichnet, dass** für jeden Satz eine erste leitfähige Spur (140A; 140B; 140C), die in der Anordnung leitfähiger Spuren eingeschlossen ist, konfiguriert ist, um eine der Elektroden des ersten Schenkels (134) mit einer entsprechenden Elektrode des zweiten Schenkels (136) zu verbinden, und eine zweite leitfähige Spur (142A; 142B; 142C), die in der Anordnung leitfähiger Spuren eingeschlossen ist, konfiguriert ist, um die entsprechende Elektrode des zweiten Schenkels (136) mit einer entsprechenden Elektrode des dritten Schenkels (138) zu verbinden, und **weiter dadurch gekennzeichnet, dass** für jeden Satz mindestens eine weitere leitfähige Spur (150A, 150B, 150C) in der Anordnung leitfähiger Spuren eingeschlossen und konfiguriert ist, um Elektroden innerhalb eines der ersten, zweiten und dritten Schenkels (134, 136, 138) miteinander zu verbinden.

2. Einrichtung (100) nach Anspruch 1, wobei der erste Schenkel (134) mindestens eine Reihe erster Elektroden (116A) umfasst, wobei jede Elektrode der mindestens einen Reihe erster Elektroden konfiguriert ist, um eines der aus einem jeweiligen Auslass (114) in der ersten Gruppe hervortretenden Schweißtröpfchen freizugeben und/oder zu transportieren.

3. Einrichtung (100) nach Anspruch 2, wobei der erste Schenkel (134) mindestens eine weitere Reihe erster Elektroden (116AD) umfasst, die konfiguriert ist, um Schweißtröpfchen von jeder Reihe der mindestens einen Reihe erster Elektroden (116AA, 116AB, 116AC) aufzunehmen und die Schweißtröpfchen von der mindestens einen Reihe erster Elektroden weg zu transportieren.

4. Einrichtung (100) nach Anspruch 3, wobei der erste Schenkel (134) eine zusätzliche Reihe erster Elektroden (116ADA) umfasst und wobei der erste Schenkel eine Vielzahl der weiteren Reihen erster Elektroden (116AAD, 116ABD, 116ACD) umfasst, wobei die zusätzliche Reihe erster Elektroden konfiguriert ist, um Schweißtröpfchen von jeder weiteren Reihe der Vielzahl von weiteren Reihen erster Elektroden aufzunehmen und die Schweißtröpfchen von der Vielzahl von weiteren Reihen erster Elektroden weg zu transportieren.

5. Einrichtung (100) nach einem der Ansprüche 1 bis 4, wobei der zweite Schenkel (136) mindestens eine Reihe zweiter Elektroden (116B) umfasst, wobei jede Elektrode der mindestens einen Reihe zweiter Elektroden konfiguriert ist, um eines der aus einem jeweiligen Auslass (114) in der zweiten Gruppe hervortretenden Schweißtröpfchen freizugeben und/oder zu transportieren.

6. Einrichtung (100) nach Anspruch 5, wobei der zweite Schenkel (136) mindestens eine weitere Reihe zweiter Elektroden (116BD) umfasst, die konfiguriert ist, um Schweißtröpfchen jeder Reihe der mindestens einen Reihe zweiter Elektroden (116BA, 116BB, 116BC) aufzunehmen und die Schweißtröpfchen von der mindestens einen Reihe zweiter Elektroden weg zu transportieren.

7. Einrichtung (100) nach Anspruch 6, wobei der zweite Schenkel (136) eine zusätzliche Reihe zweiter Elektroden umfasst und wobei der zweite Schenkel eine Vielzahl der weiteren Reihen zweiter Elektroden umfasst, wobei die zusätzliche Reihe zweiter Elektroden konfiguriert ist, um Schweißtröpfchen von jeder weiteren Reihe der Vielzahl weiterer Reihen zweiter Elektroden aufzunehmen und die Schweißtröpfchen von der Vielzahl weiterer Reihen zweiter Elektroden weg zu transportieren.

8. Einrichtung (100) nach einem der Ansprüche 1 bis 7, wobei der dritte Schenkel (138) mindestens eine Reihe dritter Elektroden (116C) umfasst, wobei jede Elektrode der mindestens einen Reihe dritter Elektroden konfiguriert ist, um eines der aus einem jeweiligen Auslass (114) in der dritten Gruppe hervortretenden Schweißtröpfchen freizugeben und/oder zu transportieren.

9. Einrichtung (100) nach Anspruch 8, wobei der dritte Schenkel (138) mindestens eine weitere Reihe dritter Elektroden (116CD) umfasst, die konfiguriert ist, um Schweißtröpfchen von jeder Reihe der mindestens einen Reihe dritter Elektroden (116CA, 116CB, 116CC) aufzunehmen und die Schweißtröpfchen von der mindestens einen Reihe dritter Elektroden weg zu transportieren; wobei der dritte Schenkel (138) optional eine zusätzliche Reihe dritter Elektroden umfasst und wobei der dritte Schenkel eine Vielzahl der weiteren Reihen dritter Elektroden umfasst, wobei die zusätzliche Reihe dritter Elektroden konfiguriert ist, um Schweißtröpfchen von jeder weiteren Reihe der Vielzahl weiterer Reihen dritter Elektroden aufzunehmen und die Schweißtröpfchen von der Vielzahl weiterer Reihen dritter Elektroden weg zu transportieren.

10. Einrichtung (100) nach Anspruch 1, wobei:
der erste Schenkel (134) mindestens eine Reihe erster Elektroden (116A) umfasst, wobei jede Elektrode der mindestens einen Reihe erster Elektroden konfiguriert ist, um eines der aus einem jeweiligen Auslass (114) in der ersten Gruppe hervortretenden Schweißtröpfchen freizugeben und/oder zu transportieren, wobei der erste Schenkel drei der Reihen erster Elektroden umfasst,
der zweite Schenkel (136) mindestens eine Reihe zweiter Elektroden (116B) umfasst, wobei jede Elektrode der mindestens einen Reihe zweiter Elektroden konfiguriert ist, um eines der aus einem jeweiligen Auslass (114) in der zweiten Gruppe hervortretenden Schweißtröpfchen freizugeben und/oder zu transportieren, wobei der zweite Schenkel drei der Reihen zweiter Elektroden umfasst,
der dritte Schenkel (138) mindestens eine Reihe dritter Elektroden (116C) umfasst, wobei jede Elektrode der mindestens einen Reihe dritter Elektroden konfiguriert ist, um eines der aus einem jeweiligen Auslass (114) in der dritten Gruppe hervortretenden Schweißtröpfchen freizugeben und/oder zu transportieren, wobei der dritte Schenkel drei der Reihen dritter Elektroden umfasst, und
zwei weitere leitfähige Spuren (150A, 150B, 150C) pro Schenkel die drei Reihen von Elektroden innerhalb jedes Schenkels miteinander verbinden.

11. Einrichtung (100) nach einem der Ansprüche 1 bis 10, wobei die mindestens eine Reihe erster Elektroden eine Vielzahl von Reihen erster Elektroden umfasst, wobei sich jede der Vielzahl von Reihen erster Elektroden mit einer weiteren Reihe der mindestens einen weiteren Reihe erster Elektroden relativ zueinander an unterschiedlichen Positionen entlang der einen weiteren Reihe erster Elektroden schneidet; und/oder wobei die mindestens eine Reihe zweiter Elektroden eine Vielzahl von Reihen zweiter Elektroden umfasst, wobei sich jede der Vielzahl von Reihen zweiter Elektroden mit einer weiteren Reihe der mindestens einen weiteren Reihe zweiter Elektroden relativ zueinander an unterschiedlichen Positionen entlang der einen weiteren Reihe zweiter Elektroden schneidet; und/oder wobei die mindestens eine Reihe dritter Elektroden eine Vielzahl von Reihen dritter Elektroden umfasst, wobei sich jede der Vielzahl von Reihen dritter Elektroden mit einer weiteren Reihe der mindestens einen weiteren Reihe dritter Elektroden relativ zueinander an unterschiedlichen Positionen entlang der einen weiteren Reihe dritter Elektroden schneidet.

12. Einrichtung (100) nach einem der Ansprüche 1 bis 11, die mindestens einen von einem ersten Schweißratensensor, einem zweiten Schweißratensensor und einem dritten Schweißratensensor (168) umfasst, wobei der erste Schweißratensensor stromabwärts der Auslässe (114) der ersten Gruppe angeordnet und konfiguriert ist, um eine Schweißrate aus Schweißtröpfchen zu bestimmen, die von dem ersten Schenkel (134) transportiert werden, der zweite Schweißratensensor stromabwärts der Auslässe der zweiten Gruppe angeordnet und konfiguriert ist, um eine Schweißrate aus Schweißtröpfchen zu bestimmen, die von dem zweiten Schenkel (136) transportiert werden, und der dritte Schweißratensensor stromabwärts der Auslässe der dritten Gruppe angeordnet und konfiguriert ist, um eine Schweißrate aus Schweißtröpfchen zu bestimmen, die von dem dritten Schenkel (138) transportiert werden.

13. Einrichtung (100) nach einem der Ansprüche 1 bis 12, die einen Biosensor (170) umfasst, der konfiguriert ist, um eine Schweißanalytkonzentration zu bestimmen, wobei der Biosensor angeordnet ist, um Schweißtröpfchen aufzunehmen, die durch den ersten, zweiten und dritten Schenkel (134, 136, 138) dorthin transportiert werden; wobei die Einrichtung optional weiter einen Schweißsammler stromabwärts des Biosensors umfasst.

14. Einrichtung (100) nach einem der Ansprüche 1 bis 13, die einen elektrischen Feldgenerator zum Laden und Entladen jeder der Elektroden (116) der Elektrobenetzungsanordnung derart umfasst, um jedes Schweißtröpfchen zu transportieren.

15. Tragbare Vorrichtung, die die Einrichtung (100) nach einem der Ansprüche 1 bis 14 umfasst; wobei die tragbare Vorrichtung optional eine Befestigungsanordnung umfasst, die konfiguriert ist, um Befestigung der Einrichtung an einem Körperteil derart zu ermöglichen, dass die Einlässe (104) Schweiß von der Haut des Körperteils aufnehmen.

## Revendications

1. Appareil (100) pour transporter des gouttelettes de sueur, l'appareil comprenant :
une pluralité de chambres (102) définies dans un substrat (110), chacune des chambres présentant une entrée (104) pour recevoir la sueur de la peau, et une sortie (114) agencée de telle sorte qu'une gouttelette de sueur se forme et fasse saillie de celle-ci à la suite du remplissage de la chambre avec de la sueur ;
dans lequel les sorties sont regroupées de manière radiale autour d'une partie centrale (135) de la surface du substrat pour définir au moins des premier, deuxième et troisième groupes de chambres ; et
un agencement d'électromouillage comprenant :
une pluralité d'électrodes (116), chaque électrode pouvant être chargée et déchargée électriquement, dans lequel l'agencement d'électromouillage comprend des première, deuxième et troisième branches (134, 136, 138) d'électrodes configurées pour permettre le transport des gouttelettes de sueur jusqu'à, ou vers, une région centrale du premier groupe dans le cas de la première branche, jusqu'à, ou vers, la région centrale du deuxième groupe dans le cas de la deuxième branche et jusqu'à, ou vers, la région centrale du troisième groupe dans le cas de la troisième branche par charge et décharge des électrodes ; et
un agencement de pistes conductrices (140A, 140B, 140C ; 142A, 142B, 142C ; 150A, 150B, 150C) agencées pour raccorder électriquement des électrodes des première, deuxième et troisième branches entre elles, **caractérisé en ce que** la pluralité d'électrodes (116) comprennent une pluralité d'ensembles d'électrodes, chaque ensemble comprenant des électrodes des première, deuxième et troisième branches (134, 136, 138) qui sont raccordées entre elles, **caractérisé en outre en ce que**, pour chaque ensemble, une première piste conductrice (140A ; 140B ; 140C) incluse dans l'agencement de pistes conductrices est configurée pour raccorder l'une des électrodes de la première branche (134) à une électrode correspondante de la deuxième branche (136) et une seconde piste conductrice (142A ; 142B ; 142C) incluse dans l'agencement de pistes conductrices est configurée pour raccorder ladite électrode correspondante de la deuxième branche (136) à une électrode correspondante de la troisième branche (138) et **caractérisé en outre en ce que**, pour chaque ensemble, au moins une autre piste conductrice (150A, 150B, 150C) est incluse dans l'agencement de pistes conductrices et configurée pour raccorder des électrodes dans l'une des première, deuxième et troisième branches (134, 136, 138) les unes aux autres.

2. Appareil (100) selon la revendication 1, dans lequel la première branche (134) comprend au moins une série de premières électrodes (116A), chaque électrode de la au moins une série de premières électrodes étant configurée pour libérer et/ou transporter l'une desdites gouttelettes de sueur faisant saillie depuis une sortie respective (114) dans le premier groupe.

3. Appareil (100) selon la revendication 2, dans lequel la première branche (134) comprend au moins une autre série de premières électrodes (116AD) configurée pour recevoir des gouttelettes de sueur de chaque série de la au moins une série de premières électrodes (116AA, 116AB, 116AC) et transporter lesdites gouttelettes de sueur loin de la au moins une série de premières électrodes.

4. Appareil (100) selon la revendication 3, dans lequel la première branche (134) comprend une série supplémentaire de premières électrodes (116ADA) et dans lequel la première branche comprend une pluralité de dites autres séries de premières électrodes (116AAD, 116ABD, 116ACD), la série supplémentaire de premières électrodes étant configurée pour recevoir des gouttelettes de sueur de chaque autre série de la pluralité d'autres séries de premières électrodes et transporter lesdites gouttelettes de sueur loin de la pluralité d'autres séries de premières électrodes.

5. Appareil (100) selon l'une quelconque des revendications 1 à 4, dans lequel la deuxième branche (136) comprend au moins une série de deuxièmes électrodes (116B), chaque électrode de la au moins une série de deuxièmes électrodes étant configurée pour libérer et/ou transporter l'une desdites gouttelettes de sueur faisant saillie depuis une sortie respective (114) dans le deuxième groupe.

6. Appareil (100) selon la revendication 5, dans lequel la seconde branche (136) comprend au moins une autre série de deuxièmes électrodes (116BD) configurées pour recevoir des gouttelettes de sueur de chaque série de la au moins une série de deuxièmes électrodes (116BA, 116BB, 116BC) et transporter lesdites gouttelettes de sueur loin de la au moins une série de deuxièmes électrodes.

7. Appareil (100) selon la revendication 6, dans lequel la deuxième branche (136) comprend une série supplémentaire de deuxièmes électrodes et dans lequel la deuxième branche comprend une pluralité de dites autres séries de deuxièmes électrodes, la série supplémentaire de deuxièmes électrodes étant configurée pour recevoir des gouttelettes de sueur de chaque autre série de la pluralité d'autres séries de deuxièmes électrodes et transporter lesdites gouttelettes de sueur loin de la pluralité d'autres séries de deuxièmes électrodes.

8. Appareil (100) selon l'une quelconque des revendications 1 à 7, dans lequel la troisième branche (138) comprend au moins une série de troisièmes électrodes (116C), chaque électrode de la au moins une série de troisièmes électrodes étant configurée pour libérer et/ou transporter l'une desdites gouttelettes de sueur faisant saillie depuis une sortie respective (114) dans le troisième groupe.

9. Appareil (100) selon la revendication 8, dans lequel la troisième branche (138) comprend au moins une autre série de troisièmes électrodes (116CD) configurée pour recevoir des gouttelettes de sueur de chaque série de la au moins une série de troisièmes électrodes (116CA, 116CB, 116CC) et transporter lesdites gouttelettes de sueur loin de la au moins une série de troisièmes électrodes ; éventuellement dans lequel la troisième branche (138) comprend une série supplémentaire de troisièmes électrodes et dans lequel la troisième branche comprend une pluralité de dites autres séries de troisièmes électrodes, la série supplémentaire de troisièmes électrodes étant configurée pour recevoir des gouttelettes de sueur de chaque autre série de la pluralité d'autres séries de troisièmes électrodes et transporter lesdites gouttelettes de sueur loin de la pluralité d'autres séries de troisièmes électrodes.

10. Appareil (100) selon la revendication 1, dans lequel :
la première branche (134) comprend au moins une série de premières électrodes (116A), chaque électrode de la au moins une série de premières électrodes étant configurée pour libérer et/ou transporter l'une desdites gouttelettes de sueur faisant saillie depuis une sortie respective (114) dans le premier groupe, la première branche comprenant trois électrodes de ladite série de premières électrodes,
la deuxième branche (136) comprend au moins une série de deuxièmes électrodes (116B), chaque électrode de la au moins une série de deuxièmes électrodes étant configurée pour libérer et/ou transporter l'une desdites gouttelettes de sueur faisant saillie depuis une sortie respective (114) dans le deuxième groupe, la deuxième branche comprenant trois électrodes de ladite série de deuxièmes électrodes,
la troisième branche (138) comprend au moins une série de troisièmes électrodes (116C), chaque électrode de la au moins une série de troisièmes électrodes étant configurée pour libérer et/ou transporter l'une desdites gouttelettes de sueur faisant saillie depuis une sortie respective (114) dans le troisième groupe, la troisième branche comprenant trois électrodes de ladite série de troisièmes électrodes, et
deux autres pistes conductrices (150A, 150B, 150C) par branche relient les trois séries d'électrodes de chaque branche entre elles.

11. Appareil (100) selon l'une quelconque des revendications 1 à 10, dans lequel la au moins une série de premières électrodes comprend une pluralité de séries de premières électrodes, chaque série de la pluralité de séries de premières électrodes croisant une autre série de ladite au moins une autre série de premières électrodes à différentes positions les unes par rapport aux autres le long de ladite autre série de premières électrodes; et/ou dans lequel la au moins une série de deuxièmes électrodes comprend une pluralité de séries de deuxièmes électrodes, chaque série de la pluralité de séries de deuxièmes électrodes croisant une autre série de ladite au moins une autre série de deuxièmes électrodes à différentes positions les unes par rapport aux autres le long de ladite autre série de deuxièmes électrodes ; et/ou dans lequel la au moins une série de troisièmes électrodes comprend une pluralité de séries de troisièmes électrodes, chaque série de la pluralité de séries de troisièmes électrodes croisant une autre série de ladite au moins une autre série de troisièmes électrodes à différentes positions les unes par rapport aux autres le long de ladite autre série de troisièmes électrodes.

12. Appareil (100) selon l'une quelconque des revendications 1 à 11, comprenant au moins un d'un premier capteur de taux de transpiration, d'un deuxième capteur de taux de transpiration et d'un troisième capteur de taux de transpiration (168), le premier capteur de taux de transpiration étant agencé en aval des sorties (114) du premier groupe et configuré pour déterminer un taux de transpiration à partir de gouttelettes de sueur transportées par la première branche (134), le deuxième capteur de taux de transpiration étant agencé en aval des sorties du deuxième groupe et configuré pour déterminer un taux de transpiration à partir de gouttelettes de sueur transportées par la deuxième branche (136) et le troisième capteur de taux de transpiration étant agencé en aval des sorties du troisième groupe et configuré pour déterminer un taux de transpiration à partir de gouttelettes de sueur transportées par la troisième branche (138).

13. Appareil (100) selon l'une quelconque des revendications 1 à 12, comprenant un biocapteur (170) configuré pour déterminer une concentration d'analytes de sueur, dans lequel le biocapteur est agencé pour recevoir des gouttelettes de sueur transportées vers celui-ci par les première, deuxième et troisième branches (134, 136, 138) ; éventuellement dans lequel l'appareil comprend en outre un collecteur de sueur en aval du biocapteur.

14. Appareil (100) selon l'une quelconque des revendications 1 à 13, comprenant un générateur de champ électrique pour charger et décharger chacune des électrodes (116) de l'agencement d'électromouillage de manière à transporter chaque dite gouttelette de sueur.

15. Dispositif pouvant être porté sur soi comprenant l'appareil (100) selon l'une quelconque des revendications 1 à 14 ; éventuellement dans lequel le dispositif pouvant être porté sur soi comprend un agencement de fixation configuré pour permettre la fixation de l'appareil à une partie du corps de telle sorte que lesdites entrées (104) reçoivent la sueur de la peau de ladite partie du corps.
